# EUROPEAN PATENT SPECIFICATION

(11) **EP 3 765 519 B1**
(45) Date of publication and mention of the grant of the patent: **17.01.2024**
(21) Application number: 19719623.1
(22) Date of filing: 13.03.2019
(51) Int. Cl.: A61P 35/00, A61K 35/17, A61K 39/00, C07K 16/28

(54) **ANTI-CXCR4 ANTIBODY COMBINED WITH ACTIVATED AND EXPANDED NATURAL KILLER CELLS FOR CANCER IMMUNOTHERAPY**
ANTI-CXCR4-ANTIKÖRPER IN KOMBINATION MIT AKTIVIERTEN UND EXPANDIERTEN NATÜRLICHEN KILLERZELLEN FÜR DIE KREBSIMMUNTHERAPIE
ANTICORPS ANTI-CXCR4 COMBINÉ À DES CELLULES TUEUSES NATURELLES ACTIVÉES ET DÉVELOPPÉES POUR UNE IMMUNOTHÉRAPIE ANTICANCÉREUSE

(30) Priority: 13.03.2018 US 201862642313 P
(43) Date of publication of application: 20.01.2021
(73) Proprietor: Fundación para la Investigación Biomédica del Hospital Universitario la Paz, 28046 Madrid (ES); Fundación del Sector Público Estatal Centro Nacional de Investigaciones Oncológicas Carlos III (F.S.P. CNIO), 28029 Madrid (ES)
(72) Inventor: VELA CUENCA, María, 28046 Madrid (ES); GONZÀLEZ NAVARRO, Pablo, 28046 Madrid (ES); VALENTÌN QUIROGA, Jaime, 28046 Madrid (ES); ESCUDERO LÓPEZ, Adela, 28046 Madrid (ES); FERNÁNDEZ CASANOVA, Lucía, 28029 Madrid (ES); PÉREZ MARTÍNEZ, Antonio, 28046 Madrid (ES)
(74) Representative: Mewburn Ellis LLP
(86) International application number: PCT/IB2019/052042
(87) International publication number: WO 2019/175802

(56) References cited:
- WO-A1-2017/049228
- WO-A2-2008/060367
- WO-A2-2016/077734
- ISHIKAWA TAKESHI ET AL: "Phase I clinical trial of adoptive transfer of expanded natural killer cells in combination with IgG1 antibody in patients with gastric or colorectal cancer.", INTERNATIONAL JOURNAL OF CANCER 15 06 2018, vol. 142, no. 12, 31 January 2018 (2018-01-31), pages 2599-2609, XP002791834, ISSN: 1097-0215
- CHEN YING ET AL: "Gene-modified NK-92MI cells expressing a chimeric CD16-BB-[zeta] or CD64-BB-[zeta] receptor exhibit enhanced cancer-killing ability in combination with therapeutic antibody.", ONCOTARGET 06 JUN 2017, vol. 8, no. 23, 6 June 2017 (2017-06-06), pages 37128-37139, XP002791835, ISSN: 1949-2553
- HOFER ERHARD ET AL: "Natural Killer Cell-Based Cancer Immunotherapies: From Immune Evasion to Promising Targeted Cellular Therapies.", FRONTIERS IN IMMUNOLOGY 2017, vol. 8, 2017, page 745, XP002791836, ISSN: 1664-3224
- RIKA FUJII ET AL: "A potential therapy for chordoma via antibody-dependent cell-mediated cytotoxicity employing NK or high-affinity NK cells in combination with cetuximab", JOURNAL OF NEUROSURGERY, vol. 128, no. 5, 28 July 2017 (2017-07-28) , pages 1419-1427, XP55488734, US ISSN: 0022-3085, DOI: 10.3171/2017.1.JNS162610

## Description

Throughout this application, various publications are referenced in parentheses by author name and date, or by Patent No. or Patent Publication No. Full citations for these publications may be found at the end of the specification immediately preceding the claims.

### CROSS REFERENCE TO RELATED APPLICATION

This application claims the benefit of U.S. Provisional Application No. 62/642,313, filed March 13, 2018.

### SEQUENCE LISTING

The present application contains a Sequence Listing which has been submitted electronically in ASCII format. The ASCII copy was created on March 11, 2019, is named 20190311_SEQL_13108WOPCT.txt and is 26,678 bytes in size.

### FIELD OF THE INVENTION

This invention relates to medical uses for treating a cancer in a subject comprising administering to the subject an anti-C-X-C Chemokine Receptor 4 (CXCR4) antibody (Ab) in combination with Activated and Expanded Natural Killer (NKAE) cell immunotherapy.

### BACKGROUND OF THE INVENTION

Metastasis, the spread of cancer cells from the primary tumor to surrounding tissues and to distant organs, is the primary cause of cancer morbidity and mortality, estimated to be responsible for about 90% of cancer deaths (Chaffer and Weinberg, 2011; Seyfried and Huysentruyt, 2013). Sarcomas primarily affect children and young adults, but despite aggressive treatment, nearly half of children with solid tumors such as rhabdomyosarcoma, osteosarcoma, Ewing sarcoma, and neuroblastoma have progressive disease. The prognosis is particularly poor for those patients with metastatic disease, at least two thirds of whom have disease progression (Oberlin *et al.,* 2008; Heare *et al.,* 2009; Matthay *et al.,* 2009). Therefore, new therapeutic approaches that bypass the cellular mechanisms of drug resistance are urgently needed, particularly for patients with high-risk features, such as metastatic disease.

Metastasis is a complex multistep process that includes local tissue invasion of metastatic cells, their survival in the circulation, "homing" to and extravasation in a secondary organ, and proliferation/growth at the metastatic sites (Nguyen *et al.,* 2009). Chemokines interacting with chemokine receptors expressed by metastasizing tumor cells have crucial roles in directing migrating cells towards secondary organs. This is well documented for the chemokine CXCL12 and its receptor, CXCR4. Müller and coworkers initially postulated and discovered the CXCL12-CXCR4 homing axis in cancer metastasis (Müller *et al.,* 2001). They demonstrated that CXCR4 expressing breast cancer cells preferentially migrated towards protein extracts of the lung, which expresses CXCL12 abundantly.

Disclosed herein is a novel immunotherapeutic approach for treating cancer, including metastatic cancer, comprising administering to a cancer patient an anti-CXCR4 Ab in combination with NKAE cell therapy.

CXCR4, also known as CD184, is a seven-transmembrane domain, G-protein coupled, cell surface receptor that is over-expressed in the majority of human cancers and, together with its endogenous ligand CXCL12, plays a fundamental role in cancer pathogenesis, including contributing to tumor growth and proliferation, adhesion, invasion, metastasis, angiogenesis and survival (Domanska *et al.,* 2013; Duda *et al.,* 2011; Balkwill, 2004; Pitt *et al.,* 2015; Passoro *et al.,* 2015; WO 2008/060367). The binding of CXCL12 to CXCR4 stimulates intracellular calcium flux, activates AKT and ERK signaling pathways, and up-regulates the formation of focal adhesions, which ultimately results in increased migration along gradients of locally expressed and secreted chemokines (Chatterjee *at al.,* 2014). It also leads to gene transcription that promotes cell survival and proliferation. CXCR4 is found in various tissues with predominant expression on hematopoietic lineage cells including B and T cells, monocytes, macrophages, natural killer (NK), and dendritic cells, as well as CD34⁺ bone marrow progenitor cells (Lee *et al.,* 1999).

Ulocuplumab (previously designated BMS-936564 or MDX-1338, and designated F7GL in WO 2008/060367) is a fully human IgG4 (S224P) monoclonal antibody (mAb) that specifically binds to CXCR4 expressed on the surface of a cell. Ulocuplumab comprises V_{H} and V_{L} regions comprising consecutively linked amino acids having the sequences shown as SEQ ID NOs: 33 and 37, respectively. Blockade of CXCR4 signaling by ulocuplumab may disrupt tumor-stromal interactions, sensitize tumor cells to cytotoxic agents, and reduce metastatic burden. In addition, ulocuplumab directly inhibits tumor growth and proliferation, and induces apoptosis, of CXCR4-expressing tumor cells (WO 2008/060367; WO 2013/071068; Kuhne *et al.,* 2013).

The known potential of immune cells to recognize and kill tumor cells suggests their potential role in anticancer treatment, which has been realized recently with the development of checkpoint inhibitors to enhance the cytotoxicity of killer T cells (Chen and Mellman, 2013; Lesokhin *et al.,* 2015). Natural killer (NK) cells are a sub-population of mononuclear lymphocytes, involved in immunity and in the host immune surveillance system. Their biological properties include targeting and killing cells that fail to express "self' major histocompatibility complex (MHC)/human leukocyte antigen (HLA) proteins, and/or tumor cells or other diseased cells that express ligands for activating NK receptors. The ability of these cells to precisely target and kill cancer stem cells and genotoxically altered cells without the need for prior immunization or activation, while maintaining tolerance to healthy cells, makes them appealing therapeutic effectors for all cancer forms, including metastases (Vivier *et al.,* 2011). Moreover, their combination with chemotherapeutic drugs and local irradiation has been reported to be helpful in breaking immune tolerance and creating a favorable microenvironment for adoptive cell therapy (Zheng *et al.,* 2015). However, NK cell activity is regulated by complex mechanisms that involve both stimulating and inhibitory signals, and a balance between activating and inhibitory receptors plays a role in tumor recognition and killing by NK cells and, thus, in their therapeutic efficacy. Membrane bound Killer-cell Activation Receptors (KARs) work together with Killer-cell Immunoglobulin-like Receptors (KIRs), which comprise both inhibitory and activating receptors that recognize allotypic variants of HLA class I alleles as KIR ligands (KIRL), to regulate NK cell function (Lanier, 2005; Pegram *et al.,* 2011; Long *et al.,* 2013). It has been postulated that the pattern of activating/inhibitory ligands found in a tumor may be a key element in the prediction of its response to NK cell therapy.

An increase in NK cell activation status, a decrease in suppressive signals (*e.g.,* using altered or mismatched MHC class I), or both, should result in heightened NK cell cytotoxicity. Moreover, the magnitude of NK cell cytotoxicity is directly proportional to the ratio between the number of NK cells and target cells (*i.e.,* effector/target ratio or E:T). Thus, in the context of NK cell therapy, the infusion of large numbers of preactivated, allogeneic NK cells is predicted to achieve the maximum anticancer effect. To this end, methods for specifically activating and expanding human NK cells from peripheral blood has been developed *(see, e.g.,* Imai *et al.,* 2005; Fujisaki *et al.,* 2009; Somanchi and Lee, 2016). These groups have reported that the K562 leukemia cell line genetically modified to express membrane-bound interleukin (IL)-15 and 4.1-BB ligand (K562-mb15-4.1BBL) specifically activates human NK cells and drives them into the cell cycle, leading to highly cytotoxic NK cells. Furthermore, the method for stimulating K562-mb15-4.1BBL cells under large-scale clinical-grade conditions was established using clinical GMP guidelines, generating large numbers of highly cytotoxic NKAE cells. Additionally, the data suggest that haploidentical donor NK cells may exert antitumor activity in patients with solid tumors undergoing allogeneic hematopoietic stem cell transplantation, partially based on an allograft-vs-tumor effect associated with KIR-HLA donor-recipient mismatch (Pende *et al.,* 2009; Moretta *et al.,* 2011). Accordingly, the use of NKAE cells is being actively explored in clinical trials on patients with hematologic malignancies and solid tumors *(see, e.g.,* the Clinical Trials Website, https://www.clinicaltrials.gov/ct2/results?term=activated+and+expanded+NK+cells&con d=cancer). These studies aim to demonstrate the potential of NKAE as a safe and effective therapy for the treatment of various types of tumors.

Ishikawa *et al.* (2018) describes Phase I clinical trials of adoptive transfer of expanded natural killer cells in combination with an IgG1 antibody to treat patients with gastric or colorectal cancer. Treatment consisted of trastuzumab- or cetuximab-based chemotherapy, plus adoptive NK cell therapy.

The disclosed invention relates to *in vitro* and *in vivo* preclinical studies demonstrating synergy between NKAE cell therapy and treatment with an anti-CXCR4 Ab in preventing tumor cell migration, invasion, implant and metastasis. The ability of this combination therapy to inhibit the migration and invasion of CXCR4⁺ tumor cells was analyzed *in vitro.* An orthotopic model of alveolar rhabdomyosarcoma in immunodeficient mice was used to evaluate the anti-tumoral and anti-metastatic synergistic effect *in vivo.* Additionally, CXCR4 expression was analyzed in pediatric rhabdomyosarcoma patients' tumors. Data presented herein suggest that this combination immunotherapy is effective to treat diverse cancers and prevent metastasis.

### SUMMARY OF THE INVENTION

The present disclosure provides a combination of therapeutically effective amounts of: (a) an isolated population of NK cells; and (b) an isolated Ab or an antigen-binding portion thereof that binds specifically to CXCR4 expressed on the surface of a cancer cell for use in a method for treating a subject afflicted with a cancer comprising administering to the subject the isolated population of NK cells and the isolated Ab or the antigen-binding portion thereof. In certain preferred embodiments, the population of NK cells comprises activated and expanded NK (NKAE) cells. In other preferred embodiments, the Ab or an antigen-binding portion thereof disrupts the interaction between CXCR4 and C-X-C motif chemokine 12 (CXCL12) and inhibits CXCR4/CXCL12 signaling. In further preferred embodiments, the Ab is ulocuplumab, comprising V_{H} and V_{L} regions having the amino acid sequences shown as SEQ ID NOs: 33 and 37, respectively.

In certain embodiments of the disclosed methods, the cancer is a solid tumor, including a pediatric tumor such as a rhabdomyosarcoma, osteosarcoma, neuroblastoma, retinoblastoma, or Ewing sarcoma. In other embodiments, the cancer is a hematological malignancy.

This disclosure also provides a kit for treating a subject afflicted with a cancer, the kit comprising: (a) one or more dosages ranging from about 50 to about 2000 mg of an Ab or an antigen-binding portion thereof that binds specifically to CXCR4 expressed on the surface of a cancer cell; (b) one or more dosages ranging from 10⁶ to 10¹⁴ of a population of NKAE cells; and (c) instructions for using theAb or portion thereof and the NKAE cells in any of the methods disclosed herein.

Other features and advantages of the instant invention will be apparent from the following detailed description and examples.

### BRIEF DESCRIPTION OF THE FIGURES

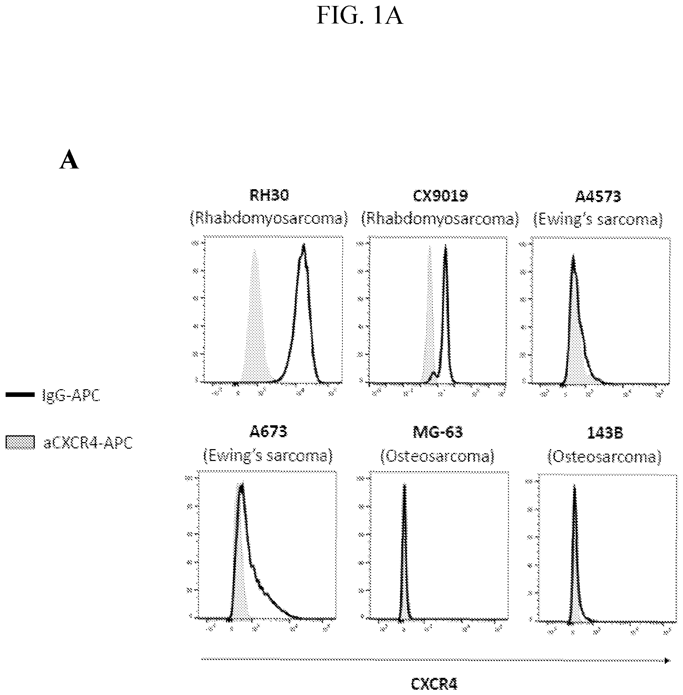

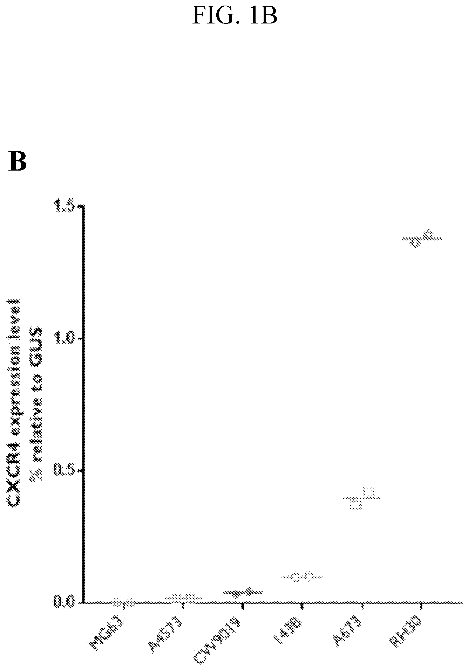

Figure 1 shows an analysis of CXCR4 expression in six sarcoma cell lines (RH30, CW9019, A4573, A673, MG-63 and 143B) by (A) flow cytometry, and (B) quantitative reverse transcriptase-polymerase chain reaction (qRT-PCR). The alveolar rhabdomyosarcoma RH30 cell line showed the highest CXCR4 expression.

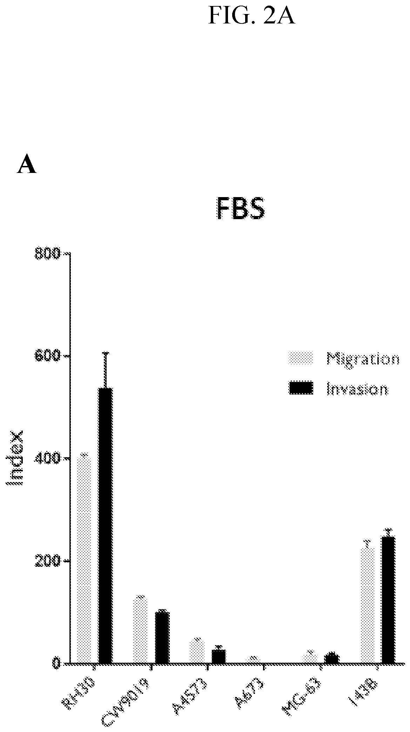

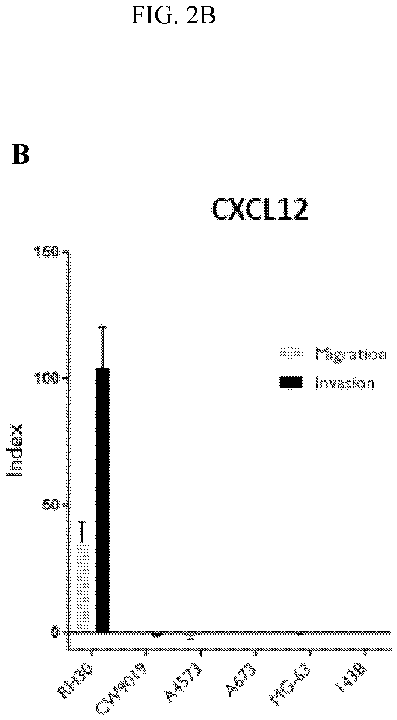

Figure 2 shows the migration and invasion capacity of the different sarcoma cell lines (RH30, CW9019, A4573, A673, MG-63 and 143B) towards (A) fetal bovine serum (FBA) or (B) CXCL12 (100 ng/ml). The RH30 cell line showed the highest migration and invasion indices towards CXCL12 (Figure 2B). Migration capacity was tested using 8 µm-pore membranes Transwell assays, and invasion capacity was measured under the same conditions using Matrigel-coated Transwell membranes.

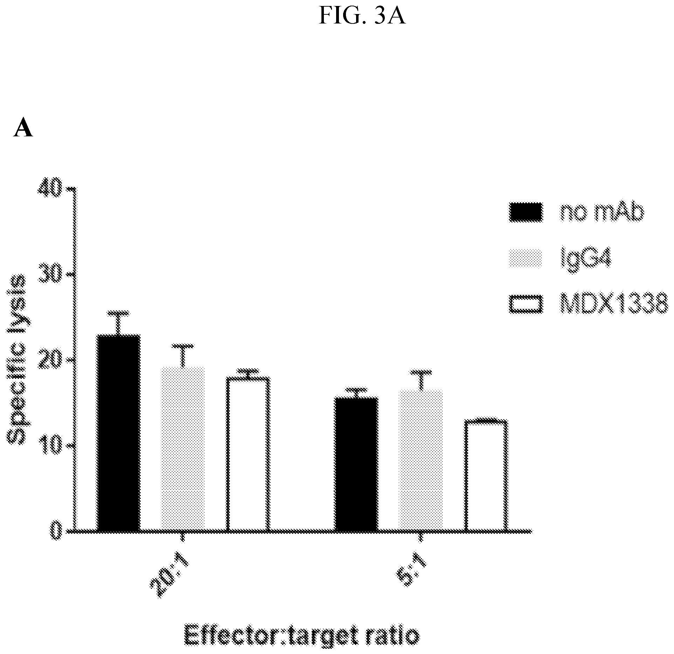

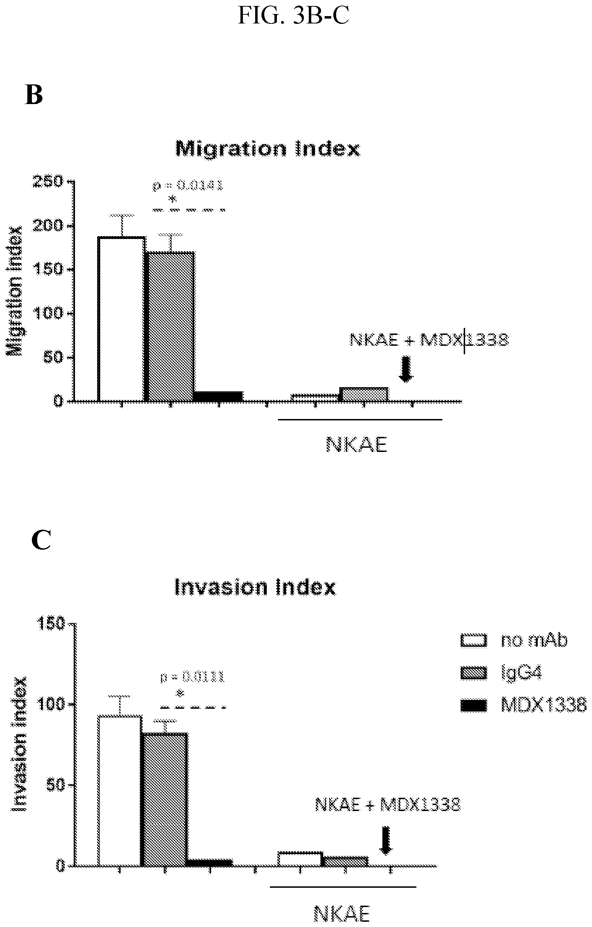

Figure 3 shows the cytotoxicity and inhibition of migration and invasion by NKAE and anti-CXCR4 therapy, individually or in combination, on rhabdomyosarcoma (RH30) cells *in vitro.* A, NKAE cells showed high cytotoxicity against RH30 cells. Specific lysis in the absence of Ab, with ulocuplumab (MDX1338; 100 µg/ml) or IgG4 control mAb (100 µg/ml) was determined at the indicated NKAE: RH30 effector:target (E:T) ratios. B-C, Ulocuplumab (MDX1338) and NKAE cells each efficiently reduced RH30 cells migration and invasion towards CXCL12, but only the combination of both agents completely abrogated it. RH30 cells migration towards a gradient of human recombinant CXCL12 chemokine was tested using Transwell plates. Ab concentration was 100 µg/ml and NKAE: RH30 E:T ratio was 5:1 (B). Invasion capacity was measured under the same conditions using Matrigel-coated Transwell membranes (C).

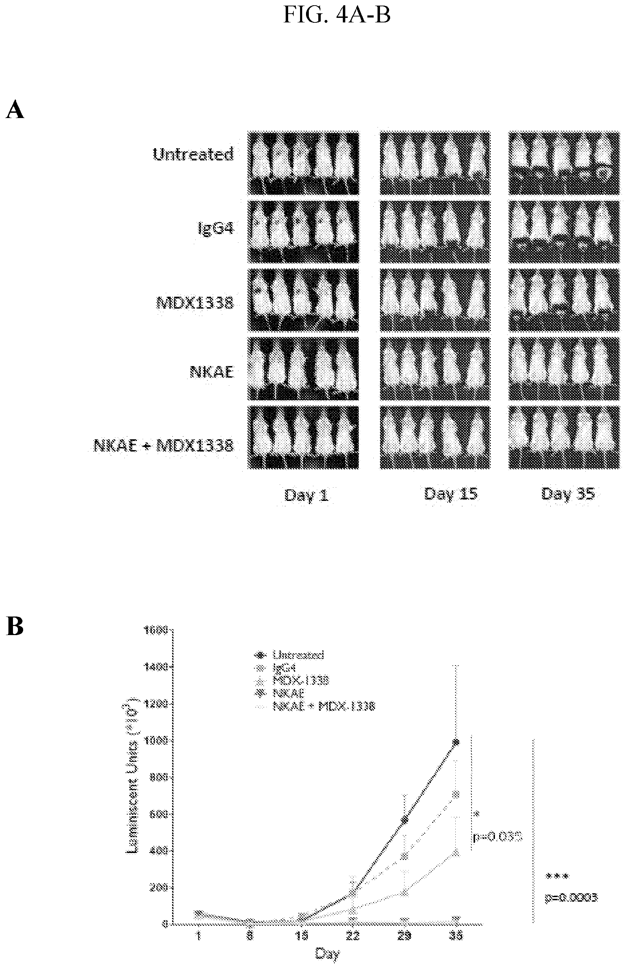

Figure 4 shows the effects of NKAE cell and anti-CXCR4 therapy, individually or in combination, on the growth of GFP⁺ Luc⁺ RH30 rhabdomyosarcoma tumor implants in the abdominal region of immunodeficient NSG mice. A, Growth of RH30 tumor implants. Five treatment arms were established: untreated; IgG4; ulocuplumab (MDX1338); NKAE cells; a combination of NKAE cells and ulocuplumab. Mice received 6 doses of mAb (15 mg/kg, twice a week), and 3 doses of NKAE (5 × 10⁶ cells, once a week). Luminescent tumors were monitored for 35 days. B, Quantification of the presence of tumor cells measured by luminescence of implanted GFP⁺ Luc⁺ RH30 cells in the treated mice. The ulocuplumab treatment alone moderately inhibited growth of the RH30 tumor implants, whereas NKAE treatment completely prevented it.

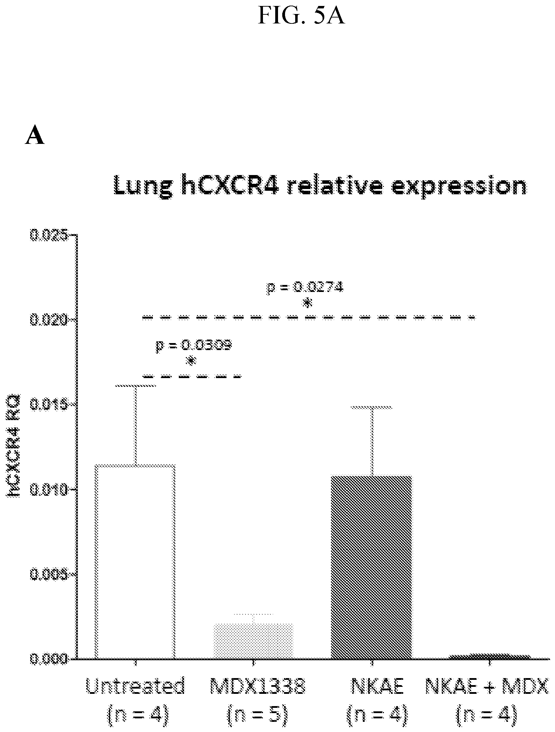

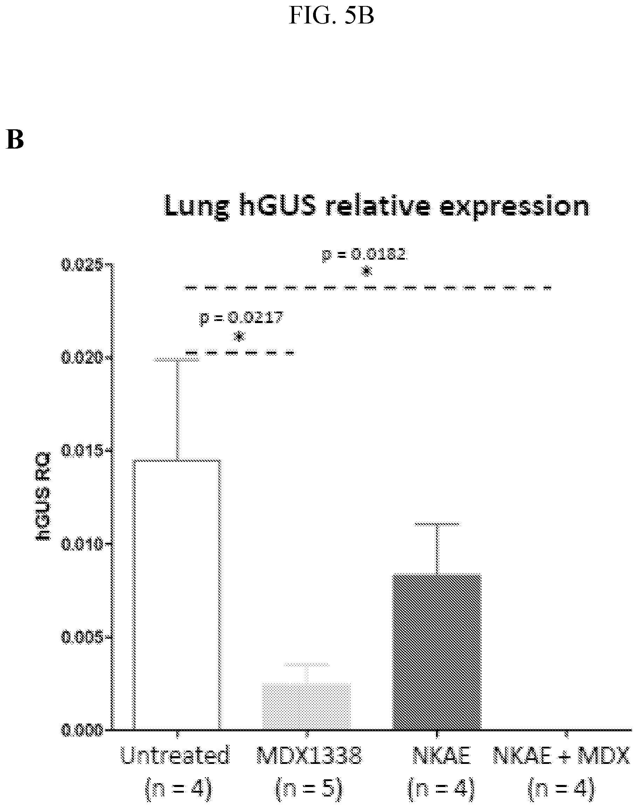

Figure 5 shows the effects of NKAE cells and ulocuplumab, individually or in combination, on the formation of lung micrometastasis from RH30 tumors in mice. A, Relative expression of human CXCR4 in RH30 lung metastatic lesions in mice measured by qRT-PCR using a human CXCR4 specific probe. Indicated values are relative to the expression of the indicated gene by a 10⁶ RH30 cells pellet. Ulocuplumab(MDX1338) reduced RH30 lung micrometastasis incidence, while the combination of ulocuplumab and NKAE completely eliminated it. B, Relative expression of human GUS in RH30 lung metastatic lesions in mice measured by qRT-PCR. The combination of both NKAE cell therapy and treatment with ulocuplumab (MDX1338) is required to completely suppress RH30 lung micrometastasis *in vivo.*

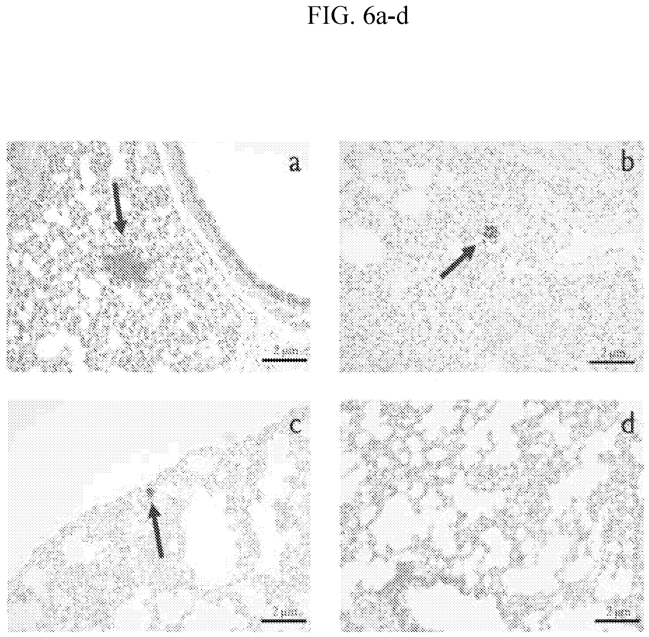

Figure 6 shows the confirmation of the inhibition of rhabdomyosarcoma RH30 micrometastases by histological methods. Lung micrometastases in untreated mice lungs were identified by hematoxylin and eosin staining (a), A*l*u sequence *in situ* hybridization (b), and CXCR4-specific mAb stain (c). Arrows identify detected micrometastases. The combination of both NKAE cell therapy and treatment with ulocuplumab is required to completely suppress RH30 lung micrometastasis *in vivo* (d).

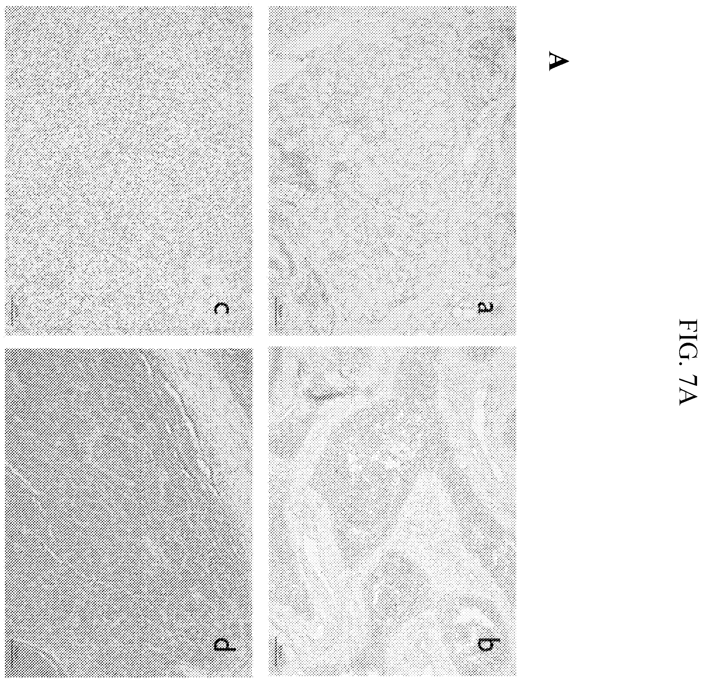

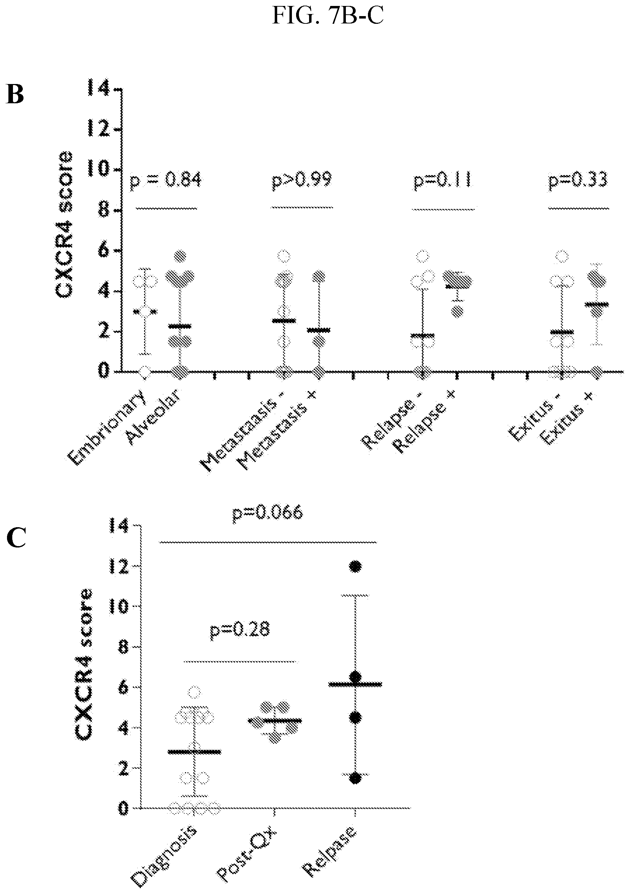

Figure 7 shows the level of CXCR4 expression on rhabdomyosarcoma patients' tumors A, Representative stainings corresponding to samples with negative (a), medium (b) and high (c) CXCR4 cytoplasmic expression. A specimen with high nuclear CXCR4 staining is also shown (d). B, CXCR4 expression score of specimens at diagnosis. C, Diagnostic versus post-chemotherapy and relapse specimens. Median ± SD is shown. Non-parametric unpaired Mann-Whitney test applied.

### DETAILED DESCRIPTION OF THE INVENTION

The present invention relates to a combination of NKAE cell immunotherapy and an anti-CXCR4 Ab for use in methods for treating cancer in a subject comprising administering the combination to the subject.

### Terms

In order that the present disclosure may be more readily understood, certain terms are first defined. As used in this application, except as otherwise expressly provided herein, each of the following terms shall have the meaning set forth below. Additional definitions are set forth throughout the application.

"Administering" refers to the physical introduction of a composition comprising a therapeutic agent to a subject, using any of the various methods and delivery systems known to those skilled in the art. A preferred route for administration of a therapeutic Ab such as an anti-CXCR4 Ab is intravenous or subcutaneous administration. Other routes of administration include intramuscular, intraperitoneal, or other parenteral routes of administration, for example by injection or infusion. The term "parenteral administration" as used herein means modes of administration other than enteral and topical administration. A preferred route for administration of NKAE cells is intravenous administration. Other routes of administration include intraarterial, intrafemoral, intraperitoneal, or intrathecal injection, intratumoral or injection into a tumor resection cavity. Administering can also be performed, for example, once, a plurality of times, and/or over one or more extended periods.

An "antibody" (Ab) shall include, without limitation, a glycoprotein immunoglobulin (Ig) which binds specifically to an antigen and comprises at least two heavy (H) chains and two light (L) chains interconnected by disulfide bonds, or an antigen-binding portion thereof. Each H chain comprises a heavy chain variable region (abbreviated herein as V*_{H}*) and a heavy chain constant region. The heavy chain constant region of an IgG Ab comprises three constant domains, C_{*H*1}, *C*_{*H*2} and C_{*H*3}. Each light chain comprises a light chain variable region (abbreviated herein as V*_{L}*) and a light chain constant region. The light chain constant region of an IgG Ab comprises one constant domain, C*_{L}*. The V*_{H}* and V*_{L}* regions can be further subdivided into regions of hypervariability, termed complementarity determining regions (CDRs), interspersed with regions that are more conserved, termed framework regions (FR). Each V*_{H}* and V*_{L}* comprises three CDRs and four FRs, arranged from amino-terminus to carboxy-terminus in the following order: FR1-CDR1-FR2-CDR2-FR3-CDR3-FR4. The variable regions of the heavy and light chains contain a binding domain that interacts with an antigen. The constant regions of the Abs may mediate the binding of the immunoglobulin to host tissues or factors, including various cells of the immune system (*e.g.,* effector cells) and the first component (C1q) of the classical complement system.

An immunoglobulin may derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the Ab class or subclass (*e.g.,* IgM, IgG1, or IgG4) that is encoded by the heavy chain constant region genes. The term "antibody" includes, by way of example, both naturally occurring and non-naturally occurring Abs; monoclonal and polyclonal Abs; chimeric and humanized Abs; human or nonhuman Abs; wholly synthetic Abs; and single chain Abs. A nonhuman Ab may be humanized partially or fully by recombinant methods to reduce its immunogenicity in man. Where not expressly stated, and unless the context indicates otherwise, the term "antibody" also includes an antigen-binding fragment or an antigen-binding portion of any of the aforementioned immunoglobulins, and includes a monovalent and a divalent fragment or portion, and a single chain Ab.

An "isolated" Ab refers to an Ab that is substantially free of other Abs having different antigenic specificities (*e.g.,* an isolated Ab that binds specifically to CXCR4 is substantially free of Abs that bind specifically to antigens other than CXCR4). An isolated Ab that binds specifically to, for example, human CXCR4 may, however, have cross-reactivity to other antigens, such as CXCR4 molecules from different species. Moreover, an "isolated" Ab may also refer to an Ab that is purified so as to be substantially free of other cellular material and/or chemicals.

The term "monoclonal" Ab (mAb) refers to a non-naturally occurring preparation of Ab molecules of single molecular composition, *i.e.,* Ab molecules whose primary sequences are essentially identical, which exhibits a single binding specificity and affinity for a particular epitope. A mAb is an example of an isolated Ab. MAbs may be produced by hybridoma, recombinant, transgenic or other techniques known to those skilled in the art.

A "chimeric" Ab refers to an Ab in which the variable regions are derived from one species and the constant regions are derived from another species, such as an Ab in which the variable regions are derived from a mouse Ab and the constant regions are derived from a human Ab.

A "human" mAb (HuMAb) refers to a mAb having variable regions in which both the framework and CDR regions are derived from human germline immunoglobulin sequences. Furthermore, if the Ab contains a constant region, the constant region also is derived from human germline immunoglobulin sequences. The human Abs of the invention may include amino acid residues not encoded by human germline immunoglobulin sequences (*e.g.,* mutations introduced by random or site-specific mutagenesis *in vitro* or by somatic mutation *in vivo*). However, the term "human" Ab, as used herein, is not intended to include Abs in which CDR sequences derived from the germline of another mammalian species, such as a mouse, have been grafted onto human framework sequences. The terms "human" Abs and "fully human" Abs are used synonymously.

A "humanized" mAb refers to a mAb in which some, most or all of the amino acids outside the CDR domains of a non-human mAb are replaced with corresponding amino acids derived from human immunoglobulins. In one embodiment of a humanized form of an Ab, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the Ab to bind to a particular antigen. A "humanized" Ab retains an antigenic specificity similar to that of the original Ab.

An "anti-antigen" Ab refers to an Ab that binds specifically to an antigen. For example, an anti-CXCR4 Ab is an Ab that binds specifically to CXCR4.

An "antigen-binding portion" of an Ab (also called an "antigen-binding fragment") refers to one or more fragments of an Ab that retain the ability to bind specifically to the antigen bound by the whole Ab.

A "cancer" refers a broad group of various diseases characterized by the uncontrolled growth of abnormal cells in the body. Unregulated cell division and growth divide and grow results in the formation of malignant tumors that invade neighboring tissues and may also metastasize to distant parts of the body through the lymphatic system or bloodstream.

"C-X-C Chemokine Receptor 4" (CXCR4; also known in the art as, for example, LESTR, Fusin or CD184) refers to a 7-transmembrane G-protein coupled receptor expressed on leukocytes, platelets and other non-hematopoietic cells that comprise the tumor stromal microenvironment. It is also over-expressed in the majority of human cancers and on Tregs and MDSCs. CXCR4 binds to a single ligand, CXCL12. The term "CXCR4" as used herein includes human CXCR4 (hCXCR4), variants, isoforms, and species homologs of hCXCR4, and analogs having at least one common epitope with hCXCR4. The complete hCXCR4 amino acid sequence can be found under GENBANK^{®} Accession No. CAA12166 and is set forth in SEQ ID NO: 43.

The term "immunotherapy" refers to the treatment of a subject afflicted with, or at risk of contracting or suffering a recurrence of, a disease by a method comprising inducing, enhancing, suppressing or otherwise modifying an immune response. "Treatment" or "therapy" of a subject refers to any type of intervention or process performed on, including the administration of an active agent to, the subject with the objective of reversing, alleviating, ameliorating, inhibiting, slowing down or preventing the onset, progression, development, severity or recurrence of a symptom, complication or condition, or biochemical indicia associated with a disease.

A "subject" includes any human or nonhuman animal. The term "nonhuman animal" includes, but is not limited to, vertebrates such as nonhuman primates, sheep, dogs, and rodents such as mice, rats and guinea pigs. In preferred embodiments, the subject is a human. The terms "subject" and "patient" are used interchangeably herein.

A "therapeutically effective amount" or "therapeutically effective dosage" of a drug or therapeutic agent is any amount of the drug or agent that, when used alone or in combination with another therapeutic agent, protects a subject against the onset of a disease or promotes disease regression evidenced by a decrease in severity of disease symptoms, an increase in frequency and duration of disease symptom-free periods, or a prevention or reduction of impairment or disability due to the disease affliction. In addition, the terms "effective" and "effectiveness" with regard to a treatment includes both pharmacological effectiveness and physiological safety. Pharmacological effectiveness refers to the ability of the drug to promote disease regression, e.g., cancer regression, in the patient. Physiological safety refers to an acceptable level of toxicity, or other adverse physiological effects at the cellular, organ and/or organism level (adverse effects) resulting from administration of the drug. The efficacy of a therapeutic agent can be evaluated using a variety of methods known to the skilled practitioner, such as by assaying the activity of the agent in *in vitro* assays, in animal model systems predictive of efficacy in humans, or in human subjects during clinical trials.

By way of example for the treatment of tumors, a therapeutically effective amount of an anti-cancer agent preferably inhibits cell growth or tumor growth by at least about 20%, more preferably by at least about 40%, even more preferably by at least about 60%, and still more preferably by at least about 80% relative to untreated subjects. In other preferred embodiments of the invention, tumor regression may be observed and continue for a period of at least about 20 days, more preferably at least about 40 days, or even more preferably at least about 60 days. Notwithstanding these ultimate measurements of therapeutic effectiveness, evaluation of immunotherapeutic drugs must also make allowance for "immune-related" response patterns.

A therapeutically effective amount of a drug includes a prophylactically effective amount, which is any amount of the drug that, when administered alone or in combination with an another therapeutic agent to a subject at risk of developing a disease (*e.g.,* a subject having a pre-malignant condition who is at risk of developing a cancer) or of suffering a recurrence of the disease, inhibits the development or recurrence of the disease (*e.g.,* a cancer). In preferred embodiments, the prophylactically effective amount prevents the development or recurrence of the disease entirely. "Inhibiting" the development or recurrence of a disease means either lessening the likelihood of the disease's development or recurrence, or preventing the development or recurrence of the disease entirely.

The use of the alternative (*e.g.,* "or") should be understood to mean either one, both, or any combination thereof of the alternatives. As used herein, the indefinite articles "a" or "an" should be understood to refer to "one or more" of any recited or enumerated component.

The term "about" refers to a numeric value, composition or characteristic that is within an acceptable error range for the particular value, composition or characteristic as determined by one of ordinary skill in the art, which will depend in part on how the value, composition or characteristic is measured or determined, *i*.*e*., the limitations of the measurement system. For example, "about" can mean within 1 or within more than 1 standard deviation per the practice in the art. Alternatively, it can mean a range of plus or minus 20%, more usually a range of plus or minus 10%. When particular values, compositions or characteristics are provided in the application and claims, unless otherwise stated, the meaning of "about" should be assumed to be within an acceptable error range for that particular value, composition or characteristic. In dosing frequenceies in drug administration regimens, the terms "once about every week," "once about every two weeks," or any other similar dosing interval terms as used herein mean approximate numbers. For example, "once about every week" can include every 7 days ± one day, *i.e.,* every 6 days to every 8 days. "Once about every 2 weeks" can include every 14 days ± 3 days, *i.e.,* every 11 days to every 17 days. Similar approximations apply, for example, to once about every 3 weeks, once about every 4 weeks, or once about every month.

The term "substantially the same" or "essentially the same" refers to a sufficiently high degree of similarity between two or more numeric values, compositions or characteristics that one of skill in the art would consider the difference between these values, compositions or characteristics to be of little or no biological and/or statistical significance within the context of the property being measured. The difference between numeric values being measured may, for example, be less than about 30%, preferably less than about 20%, and more preferably less than about 10%.

As described herein, any concentration range, percentage range, ratio range or integer range is to be understood to include the value of any integer within the recited range and, when appropriate, fractions thereof (such as one tenth and one hundredth of an integer), unless otherwise indicated.

Various aspects of the invention are described in further detail in the following subsections.

### Therapeutic Uses

Sarcomas, primarily including Ewing sarcoma, osteosarcoma and soft tissue sarcomas, are a group of relatively rare mesenchymal-derived tumors (Stiller *et al.,* 2013). Despite their low incidence among human malignancies and advances in adjuvant chemotherapy and surgical-wide resection, prognosis remains poor, mainly due to the high propensity for lung metastasis, which is the leading cause of mortality in patients with sarcomas.

Chemokines and their interaction with their corresponding receptors have been shown to play a fundamental role in the mechanisms of cancer development and the appearance of metastasis (Vela *et al.,* 2015). The expression levels of chemokines and their receptors are altered in malignant cells. This is the case of CXCR4, the chemokine receptor most frequently overexpressed in tumor cells. The interaction of CXCR4 with its ligand, CXCL12, activates a cascade of cellular signaling that promotes the survival, proliferation, adhesion and migration of the tumor cells that express it. All this can lead to the relapse of the primary tumor, and an increase inits capacity to generate distant metastases in organs in which the ligand is secreted, especially bone marrow and lungs (Chatterjee *et al.,* 2014; Duda *et al.,* 2011).

The usurping of CXCL12/CXCR4 signaling pathways by tumor cells for metastasis and protection from apoptosis long ago identified the blockade of this axis as a novel opportunity for cancer therapy. A pioneering report by Müller *et al.* (2001) demonstrated the *in vivo* relevance of CXCR4 as a target for cancer therapy, linking the expression of CXCR4 in breast carcinomas with their ability to generate regional lymph node and lung metastases. These data were supported by experiments in which a neutralizing murine anti-human CXCR4 mAb, Clone 44717.111, led to a significant decrease in lung, inguinal and axillary lymph node metastases. Subsequently, similar results were obtained treating xenografts of a human non-Hodgkin lymphoma (Bertolini *et al.,* 2002) and of a primary human acute myeloid leukemia (Tavor *et al.,* 2004) with another murine anti-human CXCR4 Ab (Clone 12G5). In both models, a significant reduction on tumor progression was reported. In endometrial cancer xenografts, treatment with 12G5 mAb led to a complete inhibition of spontaneous metastases in liver and lung, and a 28-fold decrease in metastatic index in the peritoneum (Gelmini *et al.,* 2009). Interestingly, on in intratibial human osteosarcoma xenograft model, 12G5 mAb reduced metastatic spread to the lung (Brennecke *et al.,* 2014).

Adoptively transferred NK cells, mainly resting NK cells (Rubnitz *et al.,* 2010; Curti *et al.,* 2011) or IL-2-cultured NK cells (Bachanova *et al.,* 2014; Shi *et al.,* 2013; Miller *et al.,* 2005) have been used in clinical trials. The use of activated and expanded NK cells co-cultured with human-derived antigen presenting cells is an emerging alternative (Szmania *et al.,* 2015; Leivas *et al.,* 2016; Ishikawa *et al.,* 2004; Vela *et al.,* 2018). The results of these trials show that both autologous and haploidentical NKAE is a safe therapy with no serious adverse effects and is a feasible approach for the treatment of different cancer types.

Data presented herein, show that ulocuplumab, a human mAb that blocks the CXCR4/CXCL12 axis, efficiently reduces migration and invasion index of metastatic rhabdomyosarcoma RH30 cells in *in vitro* experiments but it requires the combination of ulocuplumab with NKAE cells to completely abrogate migration and invasion. Similarly, in *in vivo* experiments, the combination of ulocuplumab and NKAE therapy was required to eliminate not only the growth of implanted RH30 tumors, but also their dissemination capacity, suppressing lung micrometastasis formation. Accordingly, this disclosure provides a combination of therapeutically effective amounts of: (a) an isolated population of NK cells, preferably NKAE cells; and (b) an isolated Ab or an antigen-binding portion thereof that binds specifically to CXCR4 expressed on the surface of a cancer cell for use in a method for treating a subject afflicted with a cancer comprising administering to the subject the isolated population of NK cells and the isolated Ab or an antigen-binding portion thereof. In preferred embodiments of any of the present medical uses, the subject is a human patient.

As shown in Example 3, an anti-CXCR4 Ab (ulocuplumab) and NKAE cell therapy, administered as monotherapy, each potently reduced both the migration of RH30 alveolar rhabdomyosarcoma cells towards CXCL12 *in vitro* (Figure 3B) and the invasion capacity of these cells *in vitro* (Figure 3C). Notably, an even greater level of inhibition of migration and invasion of the RH30 cells was exhibited by the combination of anti-CXCR4 and NKAE cell therapy, *i*.*e*., anti-CXCR4 interacted synergistically with NKAE cell therapy to completely abrogate both migration and invasion (Figures 3B and C). A combination of two therapies is considered synergistic if the antitumor effect of the combination is greater than the effect observed with monotherapy using the more efficacious treatment or greater than the sum of the level of inhibition exhibited by each treatment individually. It has been demonstrated that the anti-CXCR4 Ab, ulocuplumab, directly inhibits growth and proliferation and induces apoptosis of cancer cells (WO 2008/060367; WO 2013/071068; Kuhne *et al.,* 2013), and these tumor-inhibitory properties may contribute to the efficacy of the combination therapy disclosed herein.

A similar synergistic effect between anti-CXCR4 and NKAE cell therapy was demonstrated *in vivo* (Example 4). The anti-tumor activity of an anti-CXCR4 Ab and NKAE cell therapy, administered as monotherapy or in combination, was measured in a mouse model of RH30 alveolar rhabdomyosarcoma. Ulocuplumab exhibited a moderate inhibitory effect on tumor growth whereas NKAE cell therapy completely inhibited tumor growth in this mouse tumor model (Figure 4). However, qRT-PCR analysis (Figure 5) and immunohistochemical staining and *in situ* hybridization (Figure 6) revealed RH30 lung micrometastasis in the NKAE-treated mice. NKAE therapy had only a very modest effect on lung metastasis whereas ulocuplumab had a more pronounced effect (Figure 5), but the combination of both therapies exhibited a strong synergistic effect in completely eliminated lung metastasis (Figures 5 and 6).

### NK cell population

Various therapeutic and vaccine strategies have been proposed based on a modulation of NK cell activity. However, NK cell activity is regulated by complex mechanisms that involve both stimulating and inhibitory signals. An important set of regulatory receptors is the HLA class I-restricted, Killer-cell Immunoglobulin-like Receptor (KIR) family which comprises both inhibitory and activating family members that recognize allotypic variants of HLA class I alleles as KIR ligands (KIRL). The NK cells of a single individual typically express different combinations of KIRs, providing a repertoire of NK cells with different specificities for HLA class I molecules.

Membrane bound Killer-cell Activation Receptors (KARs) work together with KIRs to regulate NK cell function (Lanier, 2005). It was initially thought that there were only one KAR and one KIR (two-receptor model), but over the last decade, multiple different KARs and KIRs, such as the activating NKp46 and NKG2D receptors and the inhibitory KIR2DL receptors *(see* Table 1), have been discovered (opposing-signals model). Many human cancers express NKG2D ligands (NKG2DL) (Nausch and Cerwenka, 2008; Pérez-Martínez *et al.,* 2012; Fernández *et al.,* 2013). Indeed, it has recently been shown that the antitumor effect mediated by the NKG2D/NKG2DL interaction targets primarily tumor-initiating cells or stem tumor cells, both in multiple myeloma and sarcoma models (Fernández *et al.,* 2015). It has been postulated that the pattern of activating/inhibitory ligands found in a tumor may be a key element in the prediction of its response to NK cell therapy.

**Table 1. Summary of activating (KARs) and inhibitory (KIRs) receptors in NK cells**

| Receptor | Function | Ligands |
|---|---|---|
| KIR (CD158) | Activator or Inhibitor | MHC class I molecules |
| NKG2D (CD314) | Activator | MICA, MICB, ULBP1-6 |
| DNAM1 (CD226) | Activator | PVRL2 (CD112), PVR (CD155) |
| NKp46 (CD335) | Activator | Heparan sulfate, hemagglutinin, etc. |
| NKp44 (CD336) | Activator | Heparan sulfate, PCNC, etc. |
| NKp30 (CD337) | Activator | B7-H6, etc. |
| PD-1 (CD279) | Inhibitor | PD-L1, PD-L2 |
| 4-1BB (CD137) | Activator | 4-1BBL |

NK cells can kill leukemic cells and have been administered either in the setting of hematopoietic stem cell transplantation or after non-myeloablative immunosuppressive therapy to enhance the effect of chemotherapy in patients with acute leukemia, yielding encouraging results (Hsu *et al.,* 2005; Miller *et al.,* 2005; Rubnitz *et al.,* 2010). In Miller *et al.'*s study, 5 of 19 poor-prognosis patients with acute myeloid leukemia (AML) achieved complete remission after haploidentical NK-cell therapy. The results were even better in the study by Rubnitz *et al.* (2010), reporting 100% remission of 10 patients with intermediate and low risk AML.

NK cells can also lyse malignant non-hematopoietic cells as shown by reports indicating NK cell cytotoxicity against Ewing sarcoma, neuroblastoma, osteosarcoma and hepatocellular carcinoma cell lines *in vitro* and *in vivo* (Fernández *et al.,* 2015; Cho *et al.,* 2010; Verhoeven *et al.,* 2008; Pérez-Martínez *et al.,* 2015b; Kamiya *et al.,* 2016). Additionally, clinical data suggest that haploidentical donor NK cells may exert antitumor activity in children with solid tumors undergoing allogeneic hematopoietic stem cell transplantation partially based on an allograft-vs-tumor effect associated with KIR-HLA donor-recipient mismatch (Pérez-Martínez *et al.,* 2009; Pérez-Martínez *et al.,* 2015a).

The present methods combine the demonstrated cytotoxicity of NK cells against tumors with the blockade of CXCR4/CXCL12 signaling by an anti-CXCR4 Ab. In certain preferred embodiments of the therapeutic uses disclosed herein, the population of NK cells comprises NKAE cells. IL-2 and other cytokines, including IL-12, IL-15 and/or IL-21 can induce proliferative responses in human NK cells, but only a minor fraction sustains continued growth (London *et al.,* 1986; Lanier *et al.,* 1988). It has been shown that sustained expansions of NK cells require additional signals, such as the presence of β-lymphoblastoid cells (London *et al.,* 1986; Rabinowich *et al.,* 1991; Igarashi *et al.,* 2004). Methods that allow specific activation and expansion of human NK cells from peripheral blood has been developed, for example, methods based on the use of genetically modified K562 cells as a feeder for peripheral mononuclear cells of healthy donors allowing the expansion and activation of the NK cell population (Imai *et al.,* 2005; Fujisaki *et al.,* 2009; Somanchi and Lee, 2016).

In certain embodiments of the disclosed invention, NKAE cells are produced by coculturing peripheral blood mononuclear cells (PBMCs) from healthy donors with (i) irradiated β-lymphoblastoid cells modified to express a membrane-bound form of an activating cytokine such as interleukin-15 (IL-15) or interleukin-21 (IL-21), and (ii) interleukin-2 (IL-2). In certain preferred embodiments, the β-lymphoblastoid cells are K562-mb15-41BBL cells (Fujisaki *et al.,* 2009). The K562-mb15-41BBL cell line lacks MHC-I ligands for inhibitory KIR NK cells receptors and comprise K562 cells modified to express a membrane-bound form of the NK activating cytokine IL-15 and the activating ligand CD137 for the 4-1BB receptor on NK cells. In the experiments described herein *(see* Examples 3 and 4), NKAE cells were obtained by coculturing healthy donors' PBMCs with K562-mb15-41BBL cells (Fujisaki *et al.,* 2009) and IL-2 *(see* Example 3). In other preferred embodiments, the β-lymphoblastoid cells used to prepare NKAE cells are K562 mb.IL21 cells (Somanchi and Lee, 2016), which comprise K562 cells modified to express a membrane-bound form of IL-21.

### Anti-CXCR4 Abs suitable for use in the disclosed methods

The present invention provides a novel immunotherapeutic approach for treating cancer, including metastatic cancer, by combining anti-CXCR4 therapy with the demonstrated cytotoxicity of NK cells against tumors *in vitro* and *in vivo.* This combination therapy use employs an anti-CXCR4 Ab to block CXCR4/CXCL12 signaling, thereby disrupting tumor-stromal interactions, sensitizing sarcoma cells to the cytotoxicity of NK cells, and reducing tumor growth and metastatic burden.

CXCR4 is over-expressed in more than 23 human cancers, and its overexpression contributes to tumor growth, invasion, angiogenesis, metastasis, relapse, and therapeutic resistance. Non-limiting examples of cancer types associated with CXCR4 expression or the CXCR4/CXCL12 pathway include solid tumors such as breast (Dewan *et al.,* 2006), ovarian (Kajiyama *et al.,* 2008), prostate (Hirata *et al.,* 2007; Miki *et al.,* 2007), lung (Cavallaro, 2013; Gangadhar *et al.,* 2010), pancreatic (Billadeau *et al.,* 2006), kidney (Jones *et al.,* 2007; Pan *et al.,* 2006), oral (Ishikawa *et al.,* 2006; Onoue *et al.,* 2006), esophageal (Wu *et al.,* 2014), gastric (Han *et al.,* 2014), colorectal (Lv *et al.,* 2014), liver (Schimanski *et al.,* 2006), brain (Bian *et al.,* 2007), and thyroid cancer (De Falco *et al.,* 2007), melanoma (Scala *et al.,* 2005), rhabdomyosarcoma (Libura *et al.,* 2002), and osteosarcoma (Laverdiere *et al.,* 2005), as well as hematological malignancies such as acute lymphoblastic leukemia (Crazzolara *et al.,* 2001), acute myeloid leukemia (Kalinkovich *et al.,* 2006), multiple myeloma (Alsayed *et al.,* 2007; Azab *et al.,* 2009) and chronic myeloid leukemia (Jin *et al.,* 2008).

The interaction between CXCR4 and CXCL12 is essential for homing and maintaining hematopoietic stem cells within the BM microenvironment (Mohle *et al.,* 1998). The prognostic significance of CXCR4 in patients with bone and soft tissue sarcomas has been extensively studied. In certain situations an inverse correlation has been established between CXCR4 expression and patient prognosis or survival, and in clinical studies, CXCR4 has been associated with increased propensity for metastasis and decreased survival. In a recent meta-analysis, including 12 studies with 997 sarcoma patients, CXCR4 expression was found to be significantly associated with poor overall survival (HR 2.37, 95% CI 1.86-3.01; P < 0.001). As for clinicopathological features, CXCR4 expression was significantly associated with higher rate of metastasis (OR 6.97, 95% CI 2.28-21.31; P = 0.001) and higher tumor stage (OR 7.55, 95 % CI 1.25-45.47; P = 0.027) (Li *et al.,* 2015)). In the short cohort of pediatric rhabdomyosarcoma patients described in Example 5, there as a slight correlation between higher CXCR4 expression at diagnosis and relapse and fatal outcome. Tumor-initiating cells (TICs) are a subpopulation of chemoresistant tumor cells that have been shown to cause tumor recurrence and metastasis, and CXCR4 upregulation is one of the hallmarks of these cells (Duda *et al.,* 2011). It was consistently observed that tumors that still grow after chemotherapy and those that appear after a combined surgical and chemotherapeutic approach are those that clearly express the highest CXCR4 levels (Figure 7C). Targeting and eliminating of TICs are therefore priorities for the development of new therapeutic paradigms.

Example 1 shows that, among six different sarcoma cell lines tested, the RH30 alveolar rhabdomyosarcoma cell line showed the highest level of CXCR4 expression (Figure 1), concomitant with highest migration and invasion index towards CXCL12 (Figure 2). Metastasis occurs in 20-55% of sarcoma patients and, like other cancers (Chaffer and Weinberg, 2011; Seyfried and Huysentruyt, 2013), remains the main cause of death. Whereas the efficacy of the NKAE plus anti-CXCR4 combination has been demonstrated herein with a rhabdomyosarcoma cell line, a person skilled in the art would readily recognize that the present medical uses for treating cancer are applicable to any cancer that over-expresses CXCR4.

Anti-CXCR4 Abs suitable for use in the disclosed methods are Abs or antigen-binding portions thereof that bind specifically to CXCR4 with high specificity and affinity. In certain preferred embodiments, the Ab or antigen-binding portion thereof is a mAb or an antigen-binding portion thereof. In certain embodiments, the anti-CXCR4 Ab or antigen-binding portion thereof is a chimeric Ab, preferably a humanized Ab or, more preferably, a human Ab or a portion thereof. Such chimeric, humanized or human mAbs can be prepared and isolated by methods well known in the art, *e.g.,* as described in WO 2008/060367. In preferred embodiments, the subject is a human and the Ab or fragment thereof binds to a human CXCR4 receptor. In further preferred embodiments, the anti-CXCR4 Ab or antigen-binding portion thereof blocks the binding of CXCR4 and CXCL12, and inhibits the activity of CXCR4, *i.e.,* the Ab or antigen-binding portion thereof disrupts the interaction between CXCR4 and CXCL12 and inhibits CXCR4/CXCL12 signaling. In certain other embodiments, the anti-CXCR4 Ab or antigen-binding portion thereof induces apoptosis and/or inhibits growth of CXCR4⁺ tumor cells *in vivo* (as described in WO 2013/071068).

Anti-CXCR4 mAbs that bind specifically to CXCR4 with high affinity, specifically mAbs F7GL (ulocuplumab; also previously designated BMS-936564 and MDX-1338), F7, F9, F9GL, D1, D1GL, E2 and E2GL, have been exemplified and described in detail in WO 2008/060367. Methods of using these Abs to treat hematological malignancies are also described, for example, in WO 2008/060367, WO 2013/071068 and WO 2015/069874. Other anti-CXCR4 mAbs, that are suitable for use in the present therapeutic methods in combination with NKAE cells, have been described in, for example, WO 2008/142303, WO 2010/037831, WO 2009/140124, WO 2013/013025, and U.S. Publication No. 2015/0037328.

Anti-CXCR4 Abs suitable for use in the present methods preferably exhibit one or more of the following characteristics that are desirable for therapeutic applications: (a) binding with high affinity to human CXCR4 expressed on a cell surface; (b) inhibiting binding of SDF-1 to CXCR4; (c) inhibiting SDF-1-induced calcium flux in cells expressing CXCR4; (d) inhibiting SDF-1-induced migration of cells expressing CXCR4; (e) inhibiting capillary tube formation by human umbilical vein endothelial cells; (f) inducing apoptosis in cells expressing CXCR4; (g) inhibiting proliferation of CXCR4⁺ tumor cells *in vitro*; (h) inhibiting CXCR4⁺ tumor cell proliferation and/or inducing CXCR4⁺ tumor cell apoptosis *in vivo*; (i) inhibiting metastases of CXCR4⁺ tumor cells; and (j) increasing survival time of a CXCR4⁺ tumor-bearing subject.

The anti-CXCR4 mAbs disclosed in WO 2008/060367, which are suitable for use in the present methods, have been demonstrated to exhibit one or more of the following characteristics: (a) binding to human CXCR4 on a surface of a cell with an EC₅₀ of less than about 100 nM *(e.g.,* about 20-80 nM); (b) inhibiting binding of CXCL12 to CXCR4 with an EC₅₀ equal to or less than about 30 nM *(e.g.,* about 1-30 nM); (c) inhibiting CXCL12-induced calcium flux in cells expressing CXCR4 with an EC₅₀ equal to or less than about 1 nM *(e.g.,* about 0.1-1.0 nM); (d) inhibiting CXCL12-induced migration of cells expressing CXCR4 with an EC₅₀ equal to or less than about 20 nM *(e.g.,* about 10-20 nM); (e) inhibiting capillary tube formation by human umbilical vein endothelial cells; (f) inducing apoptosis in cells expressing CXCR4; (g) inhibiting proliferation of CXCR4⁺ tumor cells *in vitro*; (h) inhibiting CXCR4⁺ tumor cell proliferation and/or inducing CXCR4⁺ tumor cell apoptosis *in vivo*; (i) inhibiting metastases of CXCR4⁺ tumor cells; and (j) increasing survival time of a CXCR4⁺ tumor-bearing subject.

Anti-CXCR4 Abs usable in the methods of the present invention include mAbs that bind specifically to human CXCR4 expressed on a cell surface with high affinity, for example, with a K_{D} of 1 × 10⁻⁸ M or less as determined by surface plasmon resonance (SPR), preferably with a K_{D} of 5 × 10⁻⁹ M or less, and exhibit at least five, and preferably all, of the other preceding characteristics.

For example, an anti-CXCR4 Ab suitable for use in the disclosed methods of treatment (a) binds to human CXCR4 with a K_{D} of about 5 × 10⁻⁹ to 1 × 10⁻¹⁰ M, as determined by surface plasmon resonance (Biacore); (b) inhibits binding of CXCL12 to CXCR4 with an EC₅₀ of less than about 10 nM *(e.g.,* about 1-10 nM); (c) induces apoptosis in cells expressing CXCR4; (d) inhibits proliferation of CXCR4⁺ tumor cells *in vitro*; (e) inhibits CXCR4⁺ tumor cell proliferation and/or induces CXCR4⁺ tumor cell apoptosis *in vivo*; and (f) inhibits metastases of CXCR4⁺ tumor cells.

Accordingly, this disclosure provides a combination of therapeutically effective amounts of: (a) an isolated population of NKAE cells; and (b) an isolated Ab or an antigen-binding portion thereof that: (i) binds specifically to CXCR4 expressed on the surface of a cancer cell with high affinity, for example, with a K_{D} of about 5 × 10⁻⁹ M or less, (ii) inhibits binding of CXCL12 to CXCR4, for example, with an EC₅₀ of less than about 10 nM, and (iii) inhibits CXCR4/CXCL12 signaling, for example, inhibits CXCL12-induced migration of cells expressing CXCR4, for example, with an EC₅₀ equal to or less than about 20 nM for use in a method for treating a subject afflicted with a cancer. In certain embodiments, the Ab or an antigen-binding portion thereof also induces apoptosis in cells expressing CXCR4.

In certain aspects, the anti-CXCR4 Abs disclosed herein for therapeutic use comprise the heavy chain and light chain CDR1's, CDR2's and CDR3's of F7, F9, D1 or E2, or combinations thereof. The amino acid sequences of the V_{H} CDR1's of F7, F9, D1 and E2 are shown in SEQ ID NOs. 1-4, respectively. The amino acid sequences of the V_{H} CDR2's of F7, F9, D1 and E2 are shown in SEQ ID NOs. 5-8, respectively. The amino acid sequences of the V_{H} CDR3's of F7, F9, D1 and E2 are shown in SEQ ID NOs. 9-12, respectively. The amino acid sequences of the Vₖ CDR1's of F7, F9, D1 and E2 are shown in SEQ ID NOs. 13-16, respectively. The amino acid sequences of the Vₖ CDR2's of F7, F9, D1 and E2 are shown in SEQ ID NOs. 17-20, respectively. The amino acid sequences of the VₖCDR3's of F7, F9, D1 and E2 are shown in SEQ ID NOs. 21-24, respectively. The germline back-mutated "GL" variants, *i*.*e*., F7GL, F9GL, D1GL and E2GL, have the same CDRs as F7, F9, D1 and E2, respectively. The CDR regions identified throughout this disclosure were delineated using the Kabat system (Kabat *et al.,* 1991).

In certain other aspects, the anti-CXCR4 Abs disclosed herein for therapeutic use comprise:
(a) the CDR1, CDR2 and CDR3 domains in a heavy chain variable region having the sequence set forth in SEQ ID NO: 25 or 33, and the CDR1, CDR2 and CDR3 domains in a light chain variable region having the sequence set forth in SEQ ID NO: 29 or 37;
(b) the CDR1, CDR2 and CDR3 domains in a heavy chain variable region having the sequence set forth in SEQ ID NO: 26 or 34, and the CDR1, CDR2 and CDR3 domains in a light chain variable region having the sequence set forth in SEQ ID NO: 30 or 38;
(c) the CDR1, CDR2 and CDR3 domains in a heavy chain variable region having the sequence set forth in SEQ ID NO: 27 or 35, and the CDR1, CDR2 and CDR3 domains in a light chain variable region having the sequence set forth in SEQ ID NO: 31 or 39; or
(d) the CDR1, CDR2 and CDR3 domains in a heavy chain variable region having the sequence set forth in SEQ ID NO: 28 or 36, and the CDR1, CDR2 and CDR3 domains in a light chain variable region having the sequence set forth in SEQ ID NO: 32 or 40.

In other preferred embodiments, the anti-CXCR4 Abs of this disclosure comprise:
(a) a heavy chain variable region CDR1 comprising consecutively linked amino acids having the sequence set forth in SEQ ID NO: 1 or conservative modifications thereof; a heavy chain variable region CDR2 comprising consecutively linked amino acids having the sequence set forth in SEQ ID NO: 5 or conservative modifications thereof; a heavy chain variable region CDR3 comprising consecutively linked amino acids having the sequence set forth in SEQ ID NO: 9; a light chain variable region CDR1 comprising consecutively linked amino acids having the sequence set forth in SEQ ID NO: 13 or conservative modifications thereof; a light chain variable region CDR2 comprising consecutively linked amino acids having the sequence set forth in SEQ ID NO: 17 or conservative modifications thereof; and a light chain variable region CDR3 comprising consecutively linked amino acids having the sequence set forth in SEQ ID NO: 21 or conservative modifications thereof;
(b) a heavy chain variable region CDR1 comprising consecutively linked amino acids having the sequence set forth in SEQ ID NO: 2 or conservative modifications thereof; a heavy chain variable region CDR2 comprising consecutively linked amino acids having the sequence set forth in SEQ ID NO: 6 or conservative modifications thereof; a heavy chain variable region CDR3 comprising consecutively linked amino acids having the sequence set forth in SEQ ID NO: 10; a light chain variable region CDR1 comprising consecutively linked amino acids having the sequence set forth in SEQ ID NO: 14 or conservative modifications thereof; a light chain variable region CDR2 comprising consecutively linked amino acids having the sequence set forth in SEQ ID NO: 18 or conservative modifications thereof; and a light chain variable region CDR3 comprising consecutively linked amino acids having the sequence set forth in SEQ ID NO: 22 or conservative modifications thereof;
(c) a heavy chain variable region CDR1 comprising consecutively linked amino acids having the sequence set forth in SEQ ID NO: 3 or conservative modifications thereof; a heavy chain variable region CDR2 comprising consecutively linked amino acids having the sequence set forth in SEQ ID NO: 7 or conservative modifications thereof; a heavy chain variable region CDR3 comprising consecutively linked amino acids having the sequence set forth in SEQ ID NO: 11; a light chain variable region CDR1 comprising consecutively linked amino acids having the sequence set forth in SEQ ID NO: 15 or conservative modifications thereof; a light chain variable region CDR2 comprising consecutively linked amino acids having the sequence set forth in SEQ ID NO: 19 or conservative modifications thereof; and a light chain variable region CDR3 comprising consecutively linked amino acids having the sequence set forth in SEQ ID NO: 23 or conservative modifications thereof; or
(d) a heavy chain variable region CDR1 comprising consecutively linked amino acids having the sequence set forth in SEQ ID NO: 4 or conservative modifications thereof; a heavy chain variable region CDR2 comprising consecutively linked amino acids having the sequence set forth in SEQ ID NO: 8 or conservative modifications thereof; a heavy chain variable region CDR3 comprising consecutively linked amino acids having the sequence set forth in SEQ ID NO: 12; a light chain variable region CDR1 comprising consecutively linked amino acids having the sequence set forth in SEQ ID NO: 16 or conservative modifications thereof; a light chain variable region CDR2 comprising consecutively linked amino acids having the sequence set forth in SEQ ID NO: 20 or conservative modifications thereof; and a light chain variable region CDR3 comprising consecutively linked amino acids having the sequence set forth in SEQ ID NO: 24 or conservative modifications thereof.

In certain preferred embodiments, the anti-CXCR4 Ab or antigen-binding portion thereof comprises a heavy chain variable region CDR1 comprising consecutively linked amino acids having the sequence set forth in SEQ ID NO: 1, a heavy chain variable region CDR2 comprising consecutively linked amino acids having the sequence set forth in SEQ ID NO: 5, a heavy chain variable region CDR3 comprising consecutively linked amino acids having the sequence set forth in SEQ ID NO: 9, a light chain variable region CDR1 comprising consecutively linked amino acids having the sequence set forth in SEQ ID NO: 13, a light chain variable region CDR2 comprising consecutively linked amino acids having the sequence set forth in SEQ ID NO: 17, and a light chain variable region CDR3 comprising consecutively linked amino acids having the sequence set forth in SEQ ID NO: 21.

In other embodiments, the anti-CXCR4 Abs or antigen-binding portions thereof comprise:
(a) a heavy chain variable region CDR1 comprising consecutively linked amino acids having the sequence set forth in SEQ ID NO: 2; a heavy chain variable region CDR2 comprising consecutively linked amino acids having the sequence set forth in SEQ ID NO: 6; a heavy chain variable region CDR3 comprising consecutively linked amino acids having the sequence set forth in SEQ ID NO: 10; a light chain variable region CDR1 comprising consecutively linked amino acids having the sequence set forth in SEQ ID NO: 14; a light chain variable region CDR2 comprising consecutively linked amino acids having the sequence set forth in SEQ ID NO: 18; and a light chain variable region CDR3 comprising consecutively linked amino acids having the sequence set forth in SEQ ID NO: 22;
(b) a heavy chain variable region CDR1 comprising consecutively linked amino acids having the sequence set forth in SEQ ID NO: 3; a heavy chain variable region CDR2 comprising consecutively linked amino acids having the sequence set forth in SEQ ID NO: 7; a heavy chain variable region CDR3 comprising consecutively linked amino acids having the sequence set forth in SEQ ID NO: 11; a light chain variable region CDR1 comprising consecutively linked amino acids having the sequence set forth in SEQ ID NO: 15 a light chain variable region CDR2 comprising consecutively linked amino acids having the sequence set forth in SEQ ID NO: 19; and a light chain variable region CDR3 comprising consecutively linked amino acids having the sequence set forth in SEQ ID NO: 23; or
(c) a heavy chain variable region CDR1 comprising consecutively linked amino acids having the sequence set forth in SEQ ID NO: 4; a heavy chain variable region CDR2 comprising consecutively linked amino acids having the sequence set forth in SEQ ID NO: 8; a heavy chain variable region CDR3 comprising consecutively linked amino acids having the sequence set forth in SEQ ID NO: 12; a light chain variable region CDR1 comprising consecutively linked amino acids having the sequence set forth in SEQ ID NO: 16; a light chain variable region CDR2 comprising consecutively linked amino acids having the sequence set forth in SEQ ID NO: 20; and a light chain variable region CDR3 comprising consecutively linked amino acids having the sequence set forth in SEQ ID NO: 24.

In certain embodiments, the anti-CXCR4 Ab or antigen-binding portion thereof comprises the heavy and light chain variable regions of the F7GL, F7, F9GL, F9, D1GL, D1, E2GL and E2 mAbs. In certain preferred embodiments, the anti-CXCR4 Ab or antigen-binding portion thereof comprises a heavy chain variable region comprising consecutively linked amino acids having the sequence set forth in SEQ ID Nos. 25 or 33 and a light chain variable region comprising consecutively linked amino acids having the sequence set forth in SEQ ID Nos. 29 or 37.

In other embodiments, the anti-CXCR4 Abs or antigen-binding portions thereof comprise:
(a) a heavy chain variable region comprising consecutively linked amino acids having the sequence set forth in SEQ ID Nos. 25 or 33 and a light chain variable region comprising consecutively linked amino acids having the sequence set forth in SEQ ID Nos. 29 or 37;
(b); a heavy chain variable region comprising consecutively linked amino acids having the sequence set forth in SEQ ID Nos. 26 or 34 and a light chain variable region comprising consecutively linked amino acids having the sequence set forth in SEQ ID Nos. 30 or 38;
(c) a heavy chain variable region comprising consecutively linked amino acids having the sequence set forth in SEQ ID Nos. 27 or 35 and a light chain variable region comprising consecutively linked amino acids having the sequence set forth in SEQ ID Nos. 31 or 39; or
(c) a heavy chain variable region comprising consecutively linked amino acids having the sequence set forth in SEQ ID Nos. 28 or 36 and a light chain variable region comprising consecutively linked amino acids having the sequence set forth in SEQ ID Nos. 32 or 40.

In certain embodiments of the disclosed methods, the anti-CXCR4 Ab or antigen-binding portion thereof comprises a heavy chain constant region which is of a human IgG1, IgG2, IgG3, or IgG4 isotype. In further embodiments, the Ab or antigen-binding portion thereof comprises a heavy chain constant region (*e.g.,* human IgG1 or IgG3) that possesses effector functions including ADCC, ADCP and/or CDC, for example it is of a human IgG3 or, preferably, human IgG1 isotype, or comprises a mutation (*e.g.,* E333A or E333S; Idusogie *et al.,* 2001) that increases effector functions, and mediates the depletion of immunosuppressant regulatory T cells (Tregs) and myeloid-derived suppressor cells (MDSCs). These immunosuppressant cells are known to overexpress CXCR4 (WO 2016/201425), and anti-CXCR4-mediated depletion of Tregs and/or MDSCs may contribute to enhancement of an anti-tumor effect.

In certain other embodiments, the anti-CXCR4 Ab comprises a heavy chain constant region (*e.g.,* human IgG2 or, preferably, IgG4) that does not possesses effector functions. In further embodiments, the IgG4 heavy chain constant region of the anti-CXCR4 Ab or antigen-binding portion thereof contains an S228P mutation (numbered using the Kabat system; Kabat *et al.,* 1991), which replaces a serine residue in the hinge region with the proline residue normally found at the corresponding position in IgG1 isotype Abs. This mutation, which is present in ulocuplumab, prevents Fab arm exchange with endogenous IgG4 Abs, while retaining the low affinity for activating Fc receptors associated with wild-type IgG4 Abs.

In other embodiments, the Ab comprises a light chain constant region which is a human kappa or lambda constant region.

A suitable anti-CXCR4 Ab for use in the methods disclosed herein is ulocuplumab. Ulocuplumab has been evaluated in two Phase 1 clinical trials in subjects with various hematological malignancies including acute myeloid leukemia (AML), multiple myeloma (MM), chronic lymphocytic leukemia (CLL), follicular lymphoma (FL) and diffuse large B cell lymphoma (DLBCL) with a safe and tolerable profile. Efficacy data from the AML and MM cohorts has been presented and show encouraging results for the addition of ulocuplumab to standard therapy (Becker *et al.,* 2014; Ghobrial *et al.,* 2014), and Phase 1/2 clinical trials in AML and mutated CXCR4 Waldenström's macroglobulinemia (*see* patients are ongoing *(see* NCT02305563 and NCT03225716 on the Clinical Trials Website, http://www.clinicaltrials.gov). In certain embodiments, a human IgG1 variant of ulocuplumab is used.

Other suitable anti-CXCR4 Abs include, for example, the Abs designated c414H5 and c515H7 (WO 2010/037831), the Abs designated Antibody I, Antibody II, Antibody III, Antibody IV, and Antibody V (U.S. Patent No. 7,892,546), the Ab designated 6C7 (WO 2013/013025), and humanized 3G10 Abs, *e.g.,* the Abs designated h3G1 0.A57.A58, h3G10.1.91.A58A and h3G10.1.91.A58B (U.S. Publication No. 2015/0037328).

Anti-CXCR4 Abs usable in the methods of the disclosed invention also include antigen-binding portions of the above Abs. It has been amply demonstrated that the antigen-binding function of an Ab can be performed by fragments of a full-length Ab. Examples of binding fragments encompassed within the term "antigen-binding portion" of an Ab include (i) a Fab fragment, a monovalent fragment consisting of the V*_{L}*, V*_{H}*, *C_{L}* and C_{*H*1} domains; (ii) a F(ab')₂ fragment, a bivalent fragment comprising two Fab fragments linked by a disulfide bridge at the hinge region; (iii) a Fd fragment consisting of the V*_{H}* and C_{*H*1} domains; and (iv) a Fv fragment consisting of the V*_{L}* and V*_{H}* domains of a single arm of an Ab.

These fragments, obtained initially through proteolysis with enzymes such as papain and pepsin, have been subsequently engineered into monovalent and multivalent antigen-binding fragments. For example, although the two domains of the Fv fragment, V*_{L}* and V*_{H}*, are coded for by separate genes, they can be joined, using recombinant methods, by a synthetic linker peptide that enables them to be made as a single protein chain in which the V*_{L}* and V*_{H}* regions pair to form monovalent molecules known as single chain variable fragments (scFv). Divalent or bivalent scFvs (di-scFvs or bi-scFvs) can be engineered by linking two scFvs in within a single peptide chain known as a tandem scFv which contains two V*_{H}* and two V_{L} regions. ScFv dimers and higher multimers can also be created using linker peptides of fewer than 10 amino acids that are too short for the two variable regions to fold together, which forces the scFvs to dimerize and produce diabodies or form other multimers. Diabodies have been shown to bind to their cognate antigen with much higher affinity than the corresponding scFvs, having dissociation constants up to 40-fold lower than the K_{D} values for the scFvs. Very short linkers (≤ 3 amino acids) lead to the formation of trivalent triabodies or tetravalent tetrabodies that exhibit even higher affinities for to their antigens than diabodies. Other variants include minibodies, which are scFv-C_{*H*3} dimers, and larger scFv-Fc fragments (scFv-C_{*H*2}-C_{*H*3} dimers), and even an isolated CDR may exhibit antigen-binding function.

Accordingly, in certain embodiments, the Ab fragment is selected from a Fab, F(ab')₂, Fd and Fv fragment, a single domain Ab (sdAb), a single-chain variable fragment (scFv), a divalent scFv (di-scFv) and bivalent scFv (bi-scFv), a diabody, a minibody, and an isolated CDR. In certain preferred embodiments, the Ab fragment is selected from a Fab, F(ab')₂, Fd and Fv fragment and a single chain variable fragment (scFv). These Ab fragments are engineered using conventional recombinant techniques known to those of skill in the art, and the fragments are screened for utility in the same manner as are intact Abs. All of the above proteolytic and engineered fragments of Abs and related variants *(see* Hollinger and Hudson, 2005; Olafsen and Wu, 2010, for further details) are intended to be encompassed within the term "antigen-binding portion" of an Ab.

### Cross-competing Abs

Additional anti-CXCR4 Abs usable in the disclosed methods include Abs that bind specifically to human CXCR4 and cross-compete for binding to human CXCR4 with a reference Ab, for example, ulocuplumab (F7GL) or any of the Abs designated F7, F9, D1 and E2 *(see, e.g.,* WO 2008/060367; WO 2013/071068). The ability of a pair of Abs to "cross-compete" for binding to an antigen indicates that a first Ab binds to substantially the same epitope region of the antigen as, and reduces the binding of, a second Ab to that particular epitope region and, conversely, the second Ab binds to substantially the same epitope region of the antigen as, and reduces the binding of, the first Ab to that epitope region. Thus, the ability of a test Ab to competitively inhibit the binding of, for example, ulocuplumab to humanCXCR4, demonstrates that the test Ab binds to substantially the same epitope region of human CXCR4 as does ulocuplumab. Accordingly, anti-CXCR4 Abs usable in the disclosed methods include Abs that bind specifically to substantially the same epitope region of the antigen as a reference Ab, for example, ulocuplumab.

A first Ab is considered to bind to "substantially the same epitope" or "substantially the same determinant" as does a second Ab if the first Ab reduces the binding of the second Ab to an antigen by at least about 40%. Preferably, the first Ab reduces the binding of the second Ab to the antigen by more than about 50% *(e.g.,* at least about 60% or at least about 70%). In more preferred embodiments, the first Ab reduces the binding of the second Ab to the antigen by more than about 70% *(e.g.,* at least about 80%, at least about 90%, or about 100%). The order of the first and second Abs can be reversed, *i.e.* the "second" Ab can be first bound to the surface and the "first" is thereafter brought into contact with the surface in the presence of the "second" Ab. The Abs are considered to "cross-compete" if a competitive reduction in binding to the antigen is observed irrespective of the order in which the Abs are added to the antigen.

Cross-competing Abs are expected to have similar functional properties by virtue of their binding to substantially the same epitope region of an antigen such as a CXCR4 receptor. The higher the degree of cross-competition, the more similar will the functional properties be. For example, two cross-competing Abs are expected to have essentially the same functional properties if they each inhibit binding of the other to an epitope by at least about 80%. This similarity in function is expected to be even closer if the cross-competing Abs exhibit similar affinities for binding to the epitope as measured by the dissociation constant (K_{D}).

Cross-competing anti-antigen Abs can be readily identified based on their ability to detectably compete in standard antigen binding assays, including surface plasmon resonance (BIAcore^{®}) analysis, ELISA assays or flow cytometry, using either recombinant antigen molecules or cell-surface expressed antigen molecules. By way of example, a simple competition assay to identify whether a test Ab competes with ulocuplumab for binding to human CXCR4 may involve: (1) measuring the binding of ulocuplumab, applied at saturating concentration, to a BIAcore chip (or other suitable medium for surface plasmon resonance analysis) onto which human CXCR4 is immobilized, and (2) measuring the binding of ulocuplumab to a human CXCR4-coated BIAcore chip (or other medium suitable) to which the test Ab has been previously bound. The binding of ulocuplumab to the CXCR4-coated surface in the presence and absence of the test Ab is compared. A significant (*e.g.,* more than about 40%) reduction in binding of ulocuplumab in the presence of the test Ab indicates that both Abs recognize substantially the same epitope such that they compete for binding to the CXCR4 target. The percentage by which the binding of a first Ab to an antigen is inhibited by a second Ab can be calculated as: [1-(detected binding of first Ab in presence of second Ab)/(detected binding of first Ab in absence of second Ab)] × 100. To determine whether the Abs cross-compete, the competitive binding assay is repeated except that the binding of the test Ab to the CXCR4-coated chip in the presence of ulocuplumab is measured.

In one aspect, anti-CXCR4 Abs for use in the methods of this disclosure cross-compete for binding to CXCR4 with any of mAb F7GL (ulocuplumab; having V_{H} and V_{L} sequences as shown in SEQ ID Nos. 33 and 37, respectively), F7, having V_{H} and V_{L} sequences as shown in SEQ ID Nos. 25 and 29, respectively), mAb 79GL (having V_{H} and V_{L} sequences as shown in SEQ ID Nos. 34 and 38, respectively), mAb F9 (having V_{H} and V_{L} sequences as shown in SEQ ID Nos. 26 and 30, respectively), mAb D1GL (having V_{H} and V_{L} sequences as shown in SEQ ID Nos. 35 and 39, respectively), mAb D1 (having V_{H} and V_{L} sequences as shown in SEQ ID Nos. 27 and 31, respectively), mAb E2GL (having V_{H} and V_{L} sequences as shown in SEQ ID Nos. 36 and 40, respectively), and mAb E2 (having V_{H} and V_{L} sequences as shown in SEQ ID Nos. 28 and 32, respectively). Abs that cross-compete for binding to CXCR4 bind to the same epitope region (i.e., the same or an overlapping epitope) on CXCR4.

In certain embodiments, the cross-competing anti-CXCR4 mAb comprises a V_{H} region comprising consecutively linked amino acids having a sequence derived from a human V_{H} 3-48 germline sequence as set forth in SEQ ID NO: 41 and/or a V_{L} region comprising consecutively linked amino acids having a sequence derived from a human V_{K} L15 germline sequence as set forth in SEQ ID NO: 42.

### Broad Spectrum of Cancers Amenable to Treatment by Disclosed Uses

Immuno-oncology, which relies on using the flexibility of the immune system to attack and destroy cancer cells, is applicable to treating a very broad range of cancers *(see, e.g.,* Guillerey *et al.,* 2016; Lowry and Zehring, 2017; Callahan *et al.,* 2016; Kamta *et al.,* 2017; Farkona *et al.,* 2016). Accordingly, the disclosed medical uses employing the administration of NK cells, preferably NKAE cells, combined with blockade of the CXCR4/CXCL12 signaling pathway is applicable to treating a wide variety of both solid and liquid tumors.

In certain embodiments, the disclosed combination therapy uses may be used to treat a cancer which is a solid tumor. In certain preferred embodiments, the solid tumor is a pediatric tumor. In further embodiments, the pediatric tumor is a rhabdomyosarcoma, osteosarcoma, neuroblastoma, retinoblastoma, or Ewing sarcoma. Despite aggressive treatment, nearly half of children with such tumors have progressive disease. The prognosis is particularly poor for those patients with metastatic disease, at least two thirds of whom have disease progression. Outcomes after recurrence are generally dismal. For patients with recurrent Ewing sarcoma, for example, the likelihood of long-term survival is currently less than 20%, and less than 10% if relapse occurs within 2 years (Leavey *et al.,* 2008). Therefore, new therapeutic approaches that bypass the cellular mechanisms of drug resistance are urgently needed, particularly for patients with high-risk features, such as metastatic or recurrent disease.

In other embodiments, the solid tumor is a cancer selected from small cell lung cancer (SCLC), non-small cell lung cancer (NSCLC), squamous NSCLC, non-squamous NSCLC, squamous cell cancer, non-small cell lung cancer (NSCLC), pancreatic cancer, pancreatic ductal adenocarcinoma (PDAC), ovarian cancer, cervical cancer, carcinoma of the fallopian tubes, uterine (endometrial) cancer, carcinoma of the endometrium, uterine sarcoma, carcinoma of the cervix, carcinoma of the vagina, carcinoma of the vulva, cancer of the urethra, cancer of the ureter, prostate cancer, metastatic castration-resistant prostate cancer (mCRPC), testicular cancer, penile cancer, bladder cancer, breast cancer, triple negative breast cancer (TNBC), male breast cancer, germ cell tumor, sarcoma, skin cancer, basal cell carcinoma, squamous cell carcinoma, Merkel cell carcinoma, bone cancer, melanoma, head and neck cancer, squamous cell carcinoma of the head and neck (SCCHN), thyroid cancer, oral cancer, mouth cancer, salivary gland cancer, throat cancer, esophageal cancer, gastrointestinal cancer, gastric cancer, cancer of the small intestine, gallbladder and bile duct cancer, colorectal cancer, colon carcinoma, rectal cancer, anal cancer, liver cancer, hepatoma, kidney cancer, renal cell carcinoma, cancer of the endocrine system, tumors of the thymus gland, thymona, cancer of the parathyroid gland, cancer of the adrenal gland, soft tissue sarcoma, mesothelioma, carcinoma of the renal pelvis, neoplasm of the central nervous system (CNS), primary CNS lymphoma, tumor angiogenesis, spinal axis tumor, brain cancer, glioma, brain stem glioma, glioblastoma, glioblastoma multiforme (GBM), neuroblastoma, pituitary adenoma, epidermoid cancer, solid tumors of childhood, pediatric sarcoma, metastatic cancer, cancer of unknown primary origin, environmentally-induced cancers, virus-related cancers, AIDS-related cancers, Kaposi's sarcoma, cancers of viral origin, advanced, refractory and/or recurrent solid tumors, and any combination of the preceding solid tumors. In certain embodiments, the cancer is an advanced, unresectable, metastatic, refractory cancer, and/or recurrent cancer.

In certain other embodiments, the present combination therapy uses may be used to treat a cancer which is a hematological malignancy. Hematological malignancies include liquid tumors derived from either of the two major blood cell lineages, *i.e.,* the myeloid cell line (which produces granulocytes, erythrocytes, thrombocytes, macrophages and mast cells) or the lymphoid cell line (which produces B, T, NK and plasma cells), including all types of leukemias, lymphomas, and myelomas. Hematological malignancies that may be treated using the present combination therapy uses include, for example, cancers selected from acute lymphoblastic leukemia (ALL), AML, CLL, chronic myelogenous leukemia (CML), Hodgkin's lymphoma (HL), non-Hodgkin's lymphomas (NHLs), MM, smoldering myeloma, monoclonal gammopathy of undetermined significance (MGUS), advanced, metastatic, refractory and/or recurrent hematological malignancies, and any combinations of said hematological malignancies.

In other embodiments, the hematological malignancy is a cancer selected from acute, chronic, lymphocytic (lymphoblastic) and/or myelogenous leukemias, such as ALL, AML, CLL, and CML; lymphomas, such as HL, NHLs, of which about 85% are B cell lymphomas, including DLBCL, FL, CLL/small lymphocytic lymphoma (SLL), mantle cell lymphoma, marginal zone B-cell lymphomas (mucosa-associated lymphoid tissue (MALT) lymphoma, nodal marginal zone B-cell lymphoma, and splenic marginal zone B-cell lymphoma), Burkitt lymphoma, lymphoplasmacytoid lymphoma (LPL; also known as Waldenström's macroglobulinemia (WM)), hairy cell lymphoma, and primary central nervous system (CNS) lymphoma, NHLs that are T cell lymphomas, including precursor T-lymphoblastic lymphoma/leukemia, T-lymphoblastic lymphoma/leukemia (T-Lbly/T-ALL), peripheral T-cell lymphomas such as cutaneous T-cell lymphoma (CTLC, *i*.*e*., mycosis fungoides, Sezary syndrome and others), adult T-cell lymphoma/leukemia, angioimmunoblastic T-cell lymphoma, extranodal natural killer/T-cell lymphoma nasal type, enteropathy-associated intestinal T-cell lymphoma (EATL), anaplastic large-cell lymphoma (ALCL), and peripheral T-cell lymphoma unspecified, acute myeloid lymphoma, lymphoplasmacytoid lymphoma, monocytoid B cell lymphoma, angiocentric lymphoma, intestinal T-cell lymphoma, primary mediastinal B-cell lymphoma, post-transplantation lymphoproliferative disorder, true histiocytic lymphoma, primary effusion lymphoma, diffuse histiocytic lymphoma (DHL), immunoblastic large cell lymphoma, and precursor B-lymphoblastic lymphoma; myelomas, such as multiple myeloma, smoldering myeloma (also called indolent myeloma), monoclonal gammopathy of undetermined significance (MGUS), solitary plasmocytoma, IgG myeloma, light chain myeloma, nonsecretory myeloma, and amyloidosis; and any combinations of said hematological malignancies. The present medical uses are also applicable to treatment of advanced, metastatic, refractory and/or recurrent hematological malignancies.

### Pharmaceutical Compositions and Dosage Regimens

Abs and NK cells used in the methods disclosed herein may be constituted in a composition, *e.g.,* a pharmaceutical composition containing an Ab or a population of NKAE cells and a pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier for a composition containing an Ab is suitable for intravenous, subcutaneous, intramuscular, parenteral, spinal or epidermal administration (*e.g.,* by injection or infusion).

An option for SC injection is based on Halozyme Therapeutics' ENHANZE^{®} drug-delivery technology, involving a co-formulation of an Ab with recombinant human hyaluronidase enzyme (rHuPH20) that removes traditional limitations on the volume of biologics and drugs that can be delivered subcutaneously due to the extracellular matrix (U.S. Patent No. 7,767,429).

Preferably, the carrier for a composition containing NK cells is suitable for intravenous, intraarterial, intraperitoneal, intrafemoral, intrathecal, or intratumoral injection, or injection into a tumor resection cavity.

A pharmaceutical composition of the invention may include one or more pharmaceutically acceptable salts, anti-oxidants, aqueous and non-aqueous carriers, and/or adjuvants such as preservatives, wetting agents, emulsifying agents and dispersing agents.

Dosage regimens are adjusted to provide the optimum desired response, *e.g.,* a maximal therapeutic response and/or minimal adverse effects. The dosage for administration of an anti-CXCR4 Ab may range from about 0.01 to about 20 mg/kg, preferably from about 1.0 to about 15 mg/kg, of the subject's body weight. For example, dosages can be about 0.1, 0.3, 1, 2, 3, 5, 10, 15 or 20 mg/kg body weight, and preferably, about 1, 3, 5, 10 or 15 mg/kg body weight. Alternatively, a fixed or flat dose, *e.g.,* about 50 to about 2000 mg of the Ab, instead of a dose based on body weight, may be administered. For example, dosages can be about 200, about 400, about 800, about 1600, or about 2000 mg, and preferably, about 400, about 800, about 1600. The NKAE cells may be administered to the patient in a dose based on absolute numbers of cells ranging from about 10⁶ to about 10¹⁴ cells. For example, cells may be administered to the subject in doses of about 1 × 10⁵, 5 × 10⁵, 1 × 10⁶, 5 × 10⁶, 1 × 10⁷, 5 × 10⁷, 1 × 10⁸, 5 × 10⁸, 1 × 10⁹, 5 × 10⁹, 1 × 10¹⁰, 5 × 10¹⁰, 1 × 10¹¹, 5 × 10¹¹, 1 × 10¹², 5 × 10¹², 1 × 10¹³, 5 × 10¹³, or 1 × 10¹⁴ cells. In preferred embodiments, NKAE cells are administered to the subject in doses of about 1 × 10⁷ to 5 × 10¹⁰ cells, preferably 1 × 10⁸ to 5 × 10⁹ cells, more preferably about 5 × 10⁸ cells. In other embodiments, NKAE cells are administered to the subject in doses of about 1 × 10⁵ to 1 × 10⁸ cells/kg body weight, preferably about 1 × 10⁶ to 5 × 10⁷ cells/kg, more preferably about 7.5 × 10⁶ cells/kg. NKAE cells may be administered multiple times to the patient. For example, the patient may receive one to four infusions, typically two or three infusions. These infusions are generally spaced at least one week apart.

The dosing schedule for an Ab is typically designed to achieve exposures that result in sustained receptor occupancy (RO) based on typical pharmacokinetic properties of the Ab. An exemplary treatment regime entails administration of the anti-CXCR4 Ab or antigen-binding portion thereof twice about every week, once about every week, once about every 2 weeks, once about every 3 weeks, once about every 4 weeks, or once about every month. In certain preferred embodiments, the anti-CXCR Ab is administered to the subject approximately weekly. In other embodiments, the Ab is administered once about every 2 weeks or once about every 3 weeks. In certain preferred embodiments, the NK cells are administered about weekly to the patient. In other embodiments, the NK cells are administered to the patient twice about every week or once about every 2 weeks. The dosage and scheduling may change during a course of treatment.

In certain embodiments of any of the methods disclosed herein, the anti-CXCR4 Ab or antigen-binding portion thereof is administered to the patient by intravenous or subcutaneous administration. In further embodiments, the NK cells and the Ab or antigen-binding portion thereof are administered sequentially to the subject. "Sequential" administration means that one of the NK cells and anti-CXCR4 Ab or antigen-binding portion thereof is administered before the other. Either therapeutic may be administered first; *i*.*e*., in certain embodiments, the NK cells are administered before the Ab or antigen-binding portion thereof, whereas in other embodiments, the anti-CXCR4 Ab or antigen-binding portion thereof is administered before the NK cells. Typically, the Ab is administered by intravenous infusion over a period of about 60 minutes.

In certain embodiments, the NK cells and the Ab or antigen-binding portion thereof are administered concurrently in separate compositions, whereas in other embodiments, the NK cells and the Ab or antigen-binding portion thereof are admixed in a single composition and administered concurrently.

In certain embodiments of any of the therapeutic uses disclosed herein, administration of the combination of the NK cells and the Ab or antigen-binding portion thereof is continued for as long as clinical benefit is observed or until unmanageable toxicity or disease progression occurs.

### Medical Uses of the Combination of NK Cells and Anti-CXCR4 Abs

This disclosure also provides a population of NK cells, preferably NKAE cells, and an anti-CXCR4 Ab or an antigen-binding portion thereof for use in combination in treating a subject afflicted with cancer. These therapeutic agents may be used in combination therapy of the full range of cancers disclosed herein. In certain preferred embodiments, the cancer is a pediatric tumor, for example, a rhabdomyosarcoma, osteosarcoma, neuroblastoma, retinoblastoma, or Ewing sarcoma.

One aspect of the disclosed invention is the combined use of a population of NK cells, preferably NKAE cells, and an anti-CXCR4 Ab or an antigen-binding portion thereof for the preparation of a medicament for treating a subject afflicted with a cancer. Uses of any such NKAE cells and anti-CXCR4 Ab in combination for the preparation of medicaments are broadly applicable to the full range of cancers disclosed herein. In certain preferred embodiments of these uses, the cancer is a pediatric tumor, for example, a rhabdomyosarcoma, osteosarcoma, neuroblastoma, retinoblastoma, or Ewing sarcoma.

This disclosure also provides medical uses of a population of NKAE cells in combination with an anti-CXCR4 Ab corresponding to all the embodiments of the methods of treatment employing this combination of therapeutic agents described herein.

### Kits

Also within the scope of the present invention are kits comprising a population of NK cells, preferably NKAE cells, and an anti-CXCR4 Ab or an antigen-binding portion thereof for therapeutic use. Kits typically include a label indicating the intended use of the contents of the kit and instructions for use. The term label includes any writing, or recorded material supplied on or with the kit, or which otherwise accompanies the kit. Accordingly, this disclosure provides a kit for use in treating a subject afflicted with a cancer, the kit comprising: (a) one or more dosages ranging from about 50 to about 2000 mg of an Ab or an antigen-binding portion thereof that binds specifically to CXCR4 expressed on the surface of a cancer cell; (b) one or more dosages ranging from about 10⁶ to about 10¹⁴ of a population of NK cells, preferably NKAE cells; and (c) instructions for using the Ab or portion thereof and the NK cells in any of the combination therapy uses disclosed herein. In certain embodiments, the Abs may be co-packaged in unit dosage form. In certain preferred embodiments for use in treating human patients, the kit comprises any one of the anti-human CXCR4 Abs disclosed herein, e.g., ulocuplumab or an Ab that competes with ulocuplumab for binding to human CXCR4.

The present invention is further illustrated by the following examples.

### EXAMPLE 1

### EXPRESSION OF CXCR4 BY DIFFERENT SARCOMA CELL LINES

Surface expression of CXCR4 was analyzed by flow cytometry on confluent cell cultures of different sarcoma cell lines: two rhabdomyosarcoma cell lines (RH30 and CW9019), two Ewing sarcoma cell lines (A4573 and A673) and two osteosarcoma cell lines (MG-63 and 143B). 143B (CRL-8303), MG-63 (CRL-1427) and A673 (CRL1598) cell lines were from the American Type Culture Collection. RH30 (ACC-489) was from the Leibniz-Institut DSMZ. A4573 and CW9019 were kind gifts from Dr. Javier Alonso (Institute of Health Carlos III) and Dr. Josep Roma (Vall d'Hebron Research Institute), respectively. For staining, 2 ×10⁵ cells/well were centrifuged in V-bottom 96-well plates and washed with phosphate-buffered saline (PBS, Lonza) containing 0.5% bovine serum albumin (BSA, Lonza), 1% FBS and 0.1% sodium azide (PBSst). Non-specific binding was blocked by pre-incubating the cells with 40 µg/ml rat IgG (Sigma; 100 µl final volume, 20 min, 4°C). Cells were incubated with anti-CXCR4-APC mAb (BD Pharmingen; clone 12G5) or isotype-matched mAb (30 min, 4°C). Samples were analyzed on a Navios cytometer (Beckman Coulter).

For qRT-PCR, cell culture RNA was obtained using RNeasy Mini Kit (Qiagen) according to the manufacturer's protocol. The RNA was quantified by measuring the absorbance of extracts at 260 nm. The relative expression level of mRNA encoding human CXCR4 was determined by qRT-PCR using GUS gene expression as internal control. cDNA was synthesized from 1 µg of total RNA with the Superscript IV First-Strand Synthesis System (Invitrogen) and the supplier's protocol. The cDNA was amplified in duplicate with primers for human CXCR4 (Hs00237052_m1, Applied Biosystems) and for human GUS (Fw: 5'-GAAAATATGTGGTTGGAGAGCTCATT-3', Rv: 5'-CCGAGTGAAGATCCCCTTTTTA-3'; Probe: 5'-[6FAM]CCAGCACTCTCGTCGGTGACTGTTCA[TAMRA]-3'; all from Sigma). Amplification (50 cycles; 95°C for 15 s, 60°C for 1 min) was monitored using Fast Start TaqMan Probe Master (Roche) labelled with 6-FAM dye and non-fluorescent quencher on the Roche LightCycler 480. Relative expression was analyzed using 2^{-ΔCT} method, where ΔCT = (Ct gene of interest - Ct internal control).

As shown in Figure 1A, the highest proportion of CXCR4⁺ cells was found in RH30 alveolar rhabdomyosarcoma cell cultures, where CXCR4 expression was detected in 98.6 ± 0.7% of cells.

These results were in agreement with the expression of CXCR4 mRNA as determined by qRT-PCR analysis. RH30 cultures showed 1.38 ± 0.014 relative to GUS control gene expression (Figure 1B).

### EXAMPLE 2

### MIGRATION AND INVASION OF DIFFERENT SARCOMA CELL LINES TOWARDS A CXCL12 GRADIENT

The migration capacity of the different sarcoma cell lines (RH30, CW9019, A4573, A673, MG-63 and 143B) towards CXCL12 (100 ng/ml) or fetal bovine serum (FBS, 10%) was tested in Transwell assays (48 h), which are considered an *in vitro* indicator of *in vivo* metastatic potential. Cells were incubated in starving buffer (DMEM-1% FBS) for 24 h. Next, 2 × 10⁵ cells were suspended in 80 µl of migration buffer [DMEM-1% human serum albumin (HSA); Grifols] and placed in the upper compartment of 96-well transwell plates (8.0 µm pore size, Neuroprobe). The lower compartment was filled with 300 µl of migration buffer alone (unconditioned medium), migration buffer with 100 ng/ml CXCL12 (R&D Systems), or migration buffer with 10% FBS. Invasion capacity was measured under the same conditions, with the upper part of the Transwell membranes coated with 20 µl of 0.2 mg/ml Matrigel (Corning).

After 48 h of incubation to allow cells to migrate/invade through the membrane, the cells remaining on the upper face of the membrane were removed using a cotton wool swab, while the cells on the lower side of the membrane were fixed with methanol and stained with crystal violet. After washing with PBS, the number of migrating/invading cells was determined using a Carl Zeiss Axiovert 200 microscope. Four fields per well were counted at 200x. Migration and invasion index was calculated according to the following formula: Index = 100 × [(no. of counted cells towards conditioned medium - no. of counted cells towards unconditioned medium)/(100 - no. of counted cells towards unconditioned medium)].

The results are shown in Figure 2. Different sarcoma cell lines showed moderate-level migration and invasion capacity towards highly chemoattractant FBS (Figure 2A) but negligible migration and invasion capacity under the conditions of the assay (Figure 2B). Concomitant with its relatively high level of CXCR4 expression, only RH30 cells were able to migrate and invade, specifically, towards CXCR4 ligand, CXCL12 (Figure 2B). The RH30 migration and invasion index towards CXCL12 was 35.23 ± 8.3 and 104.04 ± 16.4, respectively.

Statistical analyses were performed using GraphPad Prism 7 software. Statistical significance was established at p < 0.05 as evaluated by unpaired Student's t test, unless otherwise indicated. Results are shown as mean ± SEM, unless otherwise indicated.

### EXAMPLE 3

### NKAE CELS ARE CYTOTOXIC AGAINST RH30 CELLS, AND ANTI-CXCR4 IN COMBINATION WITH NKAE CELLS SYNERGISTICALLY ABROGATE MIGRATION AND INVASION CAPACITY OF RH30 CELLS IN VITRO

Because ulocuplumab is a fully human IgG4 anti-CXCR4 mAb, it is not expected to induce ADCC (Davies and Sutton, 2015). The cytolytic activity of NKAE cells was measured as the percentage of killing of RH30 cells pre-coated with ulocuplumab, with an isotype control mAb, or with no mAb. NK cells from a healthy donor were activated and expanded with irradiated K562-mb15-41BBL cells (Fujisaki *et al.,* 2009) and IL-2. Briefly, peripheral blood mononuclear cells (PBMCs) from healthy donors were isolated by centrifugation over a density gradient (Ficoll-Paque, GE Healthcare) (400 g, 30 min). The genetically modified K562mbIL15-41BBL cell line, kindly provided by Professor D. Campana (National University of Singapore), was irradiated with 100 Gy. NKAE cells were obtained by co-culturing donor's PBMCs with irradiated K562-mb15-41BBL cells in a 1:1.5 ratio plus 100 U/ml of IL-2 over 14-21 days in stem cell growth medium (SCGM, Cellgenix) supplemented with 10% human AB serum (Sigma) and 100 IU/mL IL-2 (Miltenyi). Fresh medium was added every 2-3 days to a final concentration of 1 × 10⁶ cells/ml. Percentage and phenotype of NK cells (CD3⁻, CD56⁺), T cells (CD3⁺, CD56⁻) and NKT cells (CD3⁺, CD56⁺) were monitored weekly by flow cytometry (Navios, Beckman Coulter). Labelled Abs used in this study are listed in Table 2.

**Table 2. Labeled Abs used in study.**

| Specificity | Clone | Isotype | Fluorochrome | Manufacturer |
|---|---|---|---|---|
| CD3 | UCHT1 | mIgG1 | PE/Cy7 | BioLegend |
| CD45 | J33 | mIgG1 | FITC | Beckman Coulter |
| CD56 | B159 | mIgG1 | APC | BD Biosciences |
| CXCR4 | 12G5 | mIgG2a | APC | BD Biosciences |
| Isotype control | 20102 | mIgG2a | APC | R&D Systems |

In order to study the effects of various treatments on rhabdomyosarcoma cells, bioluminescent RH30 cells (RH30-GFP-Luc) were generated by infection with a recombinant lentivirus encoding green fluorescent protein (GFP) and firefly luciferase (Luc) under the EF1a promoter and with a neomycin resistance marker (Amsbio). Infected cells expressing high GFP levels were isolated by fluorescence activated cell sorting, cloned, expanded, and used for *in vivo* bioluminescence assays. RH-30-GFP-Luc cells retained surface CXCR4 expression and exhibited growth kinetics similar to parental RH30 cells. Cells were cultured in Dulbecco's modified Eagle's medium (DMEM, Lonza) supplemented with 10% heat-inactivated fetal bovine serum (FBS, Gibco), 2 mM L-glutamine, 50 U/ml penicillin, and 50 µg/ml streptomycin (complete medium).

RH30-GFP-Luc cells were pre-incubated (30 min) with indicated mAb concentrations. NKAE and target cells were co-cultured (4 h) at a 20:1 or a 5:1 ratio in DMEM- 10% FBS, then stained with 7-AAD (10 min, 4°C) and analyzed by flow cytometry. Gating on 7-AAD-positive cells within the GFP⁺ population indicated the proportion of dead target cells. Specific killing was calculated as 100 × [(% dead target cells in sample - % spontaneous dead target clls)/(100 - % spontaneous dead target cells)]. Target cells incubated without effector cells were used to assess spontaneous cell death.

As seen in Figure 3A, NKAE cells induced 23.0 ± 1.8 specific RH30 cell killing in a 20:1 E:T ratio, while ulocuplumab and control mAbs had little or no effect on the observed cytotoxicity.

The effects of ulocuplumab (MDX1338) and NKAE cells, individually and in combination, was also tested on the migration of RH30 alveolar rhabdomyosarcoma cells towards a gradient of human recombinant CXCL12 and the invasion capacity of these cells *in vitro* was tested using Transwell plates and Matrigel. For these experiments, 10⁵ RH30 cells were mixed with the indicated final concentration of ulocuplumab, control IgG4 mAb and/or 500,000 NKAE cells (effector:target ratio, E:T = 5: 1) in a final volume of 90 µl of migration buffer and the assay done as described above.

Each of ulocuplumab and NKAE efficiently reduced *in vitro* RH30 cell migration (Figure 3B) and invasion (Figure 3C), but the combination of the two therapies interacted synergistically to completely abrogate both migration and invasion (Figures 3B and 3C). This combination of anti-CXCR4 and NKAE cell therapy is considered synergistic as the observed antitumor effect is greater than the effect observed with either anti-CXCR4 or NKAE cell therapy administered as monotherapy, and greater than the sum of the level of inhibition exhibited by each treatment individually.

### EXAMPLE 4

### ANTI-CXCR4 ENHANCES IN VIVO ANTI-TUMOR ACTIVITY OF NKAE CELLS IN METASTATIC SARCOMA MOUSE TUMOR MODEL

To assess the anti-tumor activity of NKAE cell therapy, either alone or in combination with an anti-CXCR4 Ab, an *in vivo* model of metastatic sarcoma was generated using CXCR4⁺ RH30 alveolar rhabdomyosarcoma cells, which grow as peritoneal xenografts when implanted in immunodeficient NSG^{™} mice. NSG^{™} mice (NOD.Cg-Prkdc^{scid} Il2rg^{tm1Wjl}/SzJ, Charles River Laboratories) were maintained in the Alberto Sols Biomedical Research Institute animal facility. All procedures were approved by Comunidad de Madrid Animal Protection Area (PROEX 220/16) and CSIC Ethics Committee.

NKAE cells were prepared from PBMCs using K562-mb 15-41 BBL cells as described in Example 3.

Lentiviral particles expressing GFP and luciferase were used to transduce RH30 alveolar rhabdomyosarcoma cells. Six-weeks-old female mice were inoculated intravenously (i.v.) with 0.5 × 10⁶ RH30- GFP⁺Luc⁺ cells on Day 0 to generate an *in vivo* model of metastatic sarcoma. Five treatment groups were established (5 mice/group): untreated; isotype control IgG4 mAb; anti CXCR4 ulocuplumab; NKAE cells; ulocuplumab and NKAE combination. Three doses of NKAE cells (5 × 10⁶ cells each, weekly) were administered to the mice on Days 0 (day of tumor implant), 7 and 14; and 6 doses of ulocuplumab (15 mg/kg twice per week) were administered on Days 0, 3, 7, 10, 14 and 18. The size of developing tumors as determined by luminescent signal, was measured until Day 35, when mice were sacrificed.

Briefly, D-luciferin (150 mg/kg; Perkin Elmer) was administered intraperitoneally (i.p.) 10 min before analysis. During imaging, mice were anesthetized with isoflurane in a lightproof chamber using a IVIS-Lumina II (Caliper Lifesciences). Exposure time was 100 s. Living Image v4.5.2 (Perkin Elmer) was used to quantify the data and produce pseudocolor images.

Subsequently, micrometastasis in lungs, liver, bone marrow and spleen were detected and quantified by qRT-PCR using human CXCR4-specific and human GUS TaqMAN. probes. One third of mice dissected organs were used for total RNA extraction using RNAeasy Mini kit (Qiagen) according to the manufacturer's protocol. The RNA was quantified by measuring the absorpance of extracts at 260 nm. The relative expression levels of mRNA encoding human CXCR4 and human GUS were determined by semi-quantitative RT-PCR. cDNA synthesis and human CXCR4 and human GUS amplification was performed as described in Example 1. Indicated values are relative to hCXCR4 and hGUS expression by a pellet of 10⁶ RH30 cells.

Micrometastasis was also detected by immunohistochemistry using hematoxylin-eosin staining and hCXCR4-specific Ab, and by fluorescence *in situ* hybridization using Alu-DNA as the probe. Mice dissected organs were fixed overnight in 4% paraformaldehyde (pH 7.4), washed in PBS and embedded in paraffin. Consecutive sections were cut (3 µm) and stained with hematoxylin/eosin using standard procedures. The presence of RH30 cells in the lungs was studied by immune staining using an anti-human CXCR4 Ab (R&D Systems, Clone 44716, dilution 1:50), an EnVision FLEX+ visualization system (mouse, high pH; Agilent), and counterstained with with hematoxylin/eosin. For *in situ* hybridization, antigen retrieval was first performed with low pH buffer (RiboCC, Roche) and protease III (Roche). Slides were then incubated with the probe Alu positive control probe II (Ventana, Roche 05272041001). Three stringency washes were performed and slides were incubated with an intermediate, rabbit anti-DNP. An OmniRabbit (Ventana, Roche) conjugated with horseradish peroxidase was used for visualization. The immunohistochemical reaction was developed using silver as the chromogen (Silver kit, Ventana, Roche), and nuclei were counterstained with Carazzi's hematoxylin. All images were captured on a Carl Zeiss Lab.A1 microscope with a AxioCam ERC5S digital camera (Carl Zeiss).

The effect of the treatments on the growth of the luminescent tumors are shown in Figure 4A and quantitatively in Figure 4B. As Figure 4B shows, ulocuplumab (MDX1338) alone moderately inhibited RH30 tumor implant growth compared to controls (992.8 × 10³ ± 416.0 × 10³ in untreated mice mice vs. 394.7 × 10³ ± 192.1 × 10³ in MDX1338-trated mice; p = 0.035) whereas NKAE treatment was enough to completely prevent it (p = 0.0003).

Although the RH30 peritoneal primary tumors could be easily monitored using the luminescent detection system, its sensitivity is insufficient to determine the presence of RH30 GFP⁺ Luc⁺ micrometastasis in distant organs. Therefore, the presence of human cells was assessed by qRT-PCR by amplification of human GUS or human CXCR4 mRNA in mice lungs, liver, spleen and bone marrow. Human genes expression was detected only in the mouse lungs, indicating the presence of RH30 presence in that organ (Figure 5). NKAE therapy had minimal effect on lung metastasis whereas ulocuplumab administered as monotherapy had a more pronounced effect in reducing the incidence of lung micrometastasis as measured by relative expression of hCXCR4 (Figure 5A) and hGUS (Figure 5B). For human CXCR4 relative expression levels, untreated mice showed 11.4 × 10⁻³ ± 4.7 × 10⁻³ vs. 2.0 × 10⁻³ ± 0.6 × 10⁻³ of ulocuplumab (MDX1338)-treated mice. Human GUS relative expression was 14.5 × 10⁻³ ± 5.4 × 10⁻³ in untreated mice vs. 2.5 × 10⁻³ ± 1.1 × 10⁻³ in ulocuplumab-treated mice. However, the combination of both ulocuplumab and NKAE led to undetectable levels of both human CXCR4 and human GUS genes in the analyzed mice lungs (Figures 5A and 5B), indicating that this combination completely eliminated lung metastasis. Thus, the combination of anti-CXCR4 and NKAE cell therapy shows a strong synergistic effect in inhibiting growth of RH30 alveolar rhabdomyosarcomas and in abrogating lung metastasis.

Immunohistochemical and *in situ* hybridization analysis further confirmed these results (Figure 6). Sarcoma lung micrometastases were identified in the lungs of untreated mice by hematoxylin and eosin staining (Figure 6a), *Alu* sequence hybridization (Figure 6b), and CXCR4-specific Ab staining (Figure 6c), whereas mice treated with the combination of NKAE and ulocuplumab showed an absence of micrometastasis (Figure 6d).

### EXAMPLE 5

### CXCR4 EXPRESSION IN SAMPLES OF PEDIATRIC RHABDOMYOSARCOMA PATIENTS IS HIGHER IN POST-CHEMOTHERAPY AND POST-RELAPSE SAMPLES THAN IN DIAGNOSTIC SAMPLES

The expression of CXCR4 was evaluated by immunohistochemistry in 23 pediatric rhabdomyosarcoma specimens deposited in La Paz University Hospital Biobank from 2010 to 2017. The median follow up period was 7.9 years (range 0.6-18.4) and the median age of patients was 6.6 years (range 0.2-14.1). 14 specimens were diagnostic samples, 5 specimens were collected after chemotherapeutic treatment, and 4 specimens were relapse samples. Human cell samples were obtained after informed patient consent in accordance with the Helsinki Declaration. Research was approved by the La Paz Hospital Ethics Committee for Clinical Research (PI-2295). For tumor CXCR4 immunohistochemistry, formalin-fixed, paraffin-embedded tumor slides were deparaffinized, hydrated, stained using an anti-human CXCR4 mAb (dilution 1: 10; Clone 44716; R&D Systems) and an EnVision FLEX+ visualization system (mouse, high pH; Agilent), and counterstained with hematoxylin-eosin.

The specificity of the Ab was confirmed by immunostaining of tonsil tissue. The proportion of CXCR4⁺ tumor cells was assigned a score between 0 and 5, and the staining intensity a score between 0 and 3. These 2 values were added to produce the final staining score. Given recent studies describing nuclear localization of CXCR4 in some cancers (Krook *et al.,* 2014; Li *et al.,* 2015), both cytoplasmic and nuclear staining were assessed and equally weighted. Nuclear staining of CXCR4 was present in 15% of cases in the tested series and cytoplasmic staining in 68%.

In diagnostic samples, no differences in CXCR4 expression between embryonic vs. alveolar rhabdomyosarcomas nor between samples from localized tumors vs. primary tumors that already had metastasized (Figure 7A). Regarding the outcome of those patients, a slightly higher CXCR4 score was observed in those samples from patients that subsequently relapsed and also from those patients that finally died of their disease.

Interestingly, when CXCR4 expression scores from diagnostic samples (2.8 ± 2.1) we compared with post-chemotherapy samples (4.4 ± 0.7) and relapse samples (6.1 ± 4.4), a strong trend towards higher CXCR4 expression was observed in the latter samples (Figure 7B).

### Sequence Listing Summary

| SEQ ID NO: | Description |
|---|---|
| 1 | Heavy chain CDR1 sequence of F7 |
| 2 | Heavy chain CDR1 sequence of F9 |
| 3 | Heavy chain CDR1 sequence of D1 |
| 4 | Heavy chain CDR1 sequence of E2 |
| 5 | Heavy chain CDR2 sequence of F7 |
| 6 | Heavy chain CDR2 sequence of F9 |
| 7 | Heavy chain CDR2 sequence of D1 |
| 8 | Heavy chain CDR2 sequence of E2 |
| 9 | Heavy chain CDR3 sequence of F7 |
| 10 | Heavy chain CDR3 sequence of F9 |
| 11 | Heavy chain CDR3 sequence of D1 |
| 12 | Heavy chain CDR3 sequence of E2 |
| 13 | Light chain CDR1 sequence of F7 |
| 14 | Light chain CDR1 sequence of F9 |
| 15 | Light chain CDR1 sequence of D1 |
| 16 | Light chain CDR1 sequence of E2 |
| 17 | Light chain CDR2 sequence of F7 |
| 18 | Light chain CDR2 sequence of F9 |
| 19 | Light chain CDR2 sequence of D1 |
| 20 | Light chain CDR2 sequence of E2 |
| 21 | Light chain CDR3 sequence of F7 |
| 22 | Light chain CDR3 sequence of F9 |
| 23 | Light chain CDR3 sequence of D1 |
| 24 | Light chain CDR3 sequence of E2 |
| 25 | V_{H} amino acid sequence of F7 |
| 26 | V_{H} amino acid sequence of F9 |
| 27 | V_{H} amino acid sequence of D1 |
| 28 | V_{H} amino acid sequence of E2 |
| 29 | V_{L} amino acid sequence of F7 |
| 30 | V_{L} amino acid sequence of F9 |
| 31 | V_{L} amino acid sequence of D1 |
| 32 | V_{L} amino acid sequence of E2 |
| 33 | V_{H} amino acid sequence of F7GL |
| 34 | V_{H} amino acid sequence of F9GL |
| 35 | V_{H} amino acid sequence of D1GL |
| 36 | V_{H} amino acid sequence of E2GL |
| 37 | V_{L} amino acid sequence of F7GL |
| 38 | V_{L} amino acid sequence of F9GL |
| 39 | V_{L} amino acid sequence of D1GL |
| 40 | V_{L} amino acid sequence of E2GL |
| 41 | V_{H} 3-48 germline amino acid sequence |
| 42 | V_{K} L15 germline amino acid sequence |
| 43 | Human CXCR4 amino acid sequence |

### REFERENCES

Alsayed Y, Ngo H, Runnels J, Leleu X, Singha UK et al. (2007) Mechanisms of regulation of CXCR4/SDF-1 (CXCL12)-dependent migration and homing in multiple myeloma. Blood 109(7):2708-17.
Azab AK, Runnels JM, Pitsillides C, Moreau AS, Azab F et al. (2009) CXCR4 inhibitor AMD3100 disrupts the interaction of multiple myeloma cells with the bone marrow microenvironment and enhances their sensitivity to therapy. Blood 113(18):4341-51.
Bachanova V, Cooley S, Defor TE, Verneris MR, Zhang B et al. (2014) Clearance of acute myeloid leukemia by haploidentical natural killer cells is improved using IL-2 diphtheria toxin fusion protein. Blood 123, 3855-3863.
Balkwill F (2004) The significance of cancer cell expression of the chemokine receptor CXCR4. Semin Cancer Biol 14:171-9.
Bertolini 1, Dell'Agnola C, Mancuso P et al. (2002) CXCR4 neutralization , a novel therapeutic approach for non-Hodgkin's lymphoma. 62(11):3106-12.
Bian XW, Yang SX, Chen JH et al. (2007) Preferential expression of chemokine receptor CXCR4 by highly malignant human gliomas and its association with poor patient survival. Neurosurgery 61:570-8.
Billadeau DD, Chatterjee S, Bramati P et al. (2006) Characterization of the CXCR4 signaling in pancreatic cancer cells. Int J Gastrointest Cancer 37:110-9.
Becker PS, Foran J, Altman J et al. (2014) Targeting the CXCR4 pathway: Safety, tolerability and clinical activity of BMS-936564 (ulocuplumab), an anti-CXCR4 antibody, in relapsed refractory acute myeloid leukemia. 56th American Society of Hematology (ASH) Annual Meeting, San Francisco, December 6-9, 2014, Oral Presentation No. 386.
Brennecke P, ArltMJ, Campanile C et al. (2014) CXCR4 antibody treatment suppresses metastatic spread to the lung of intratibial human osteosarcoma xenografts in mice. Clin Exp Metastasis 31, 339-49.
Callahan M, Postow MA, Wolchok JD (2016) Targeting T cell co-receptors for cancer therapy. Immunity 44(5):1069-78.
Cavallaro S (2013) CXCR4/CXCL12 in non-small-cell lung cancer metastasis to the brain. Int J Mol Sci 14:1713-27.
Chaffer CL, Weinberg RA (2011) A perspective on cancer cell metastasis. Science 331:1559-64.
Chatterjee S, Behnam Azad B, Nimmagadda S (2014) The intricate role of CXCR4 in cancer. Adv Cancer Res 124:31-82.
Chen DS, Mellman I (2013) Oncology meets immunology: the cancer-immunity cycle. Immunity 39(1):1-10.
Cho D, Shook DR, Shimasaki N (2010) Cytotoxicity of activated natural killer cells against pediatric solid tumors. Clin Cancer Res 16(15):3901-9.
Clinical Trials Website, https://www.clinicaltrials.gov/ct2/results?term=activated+and+expanded+NK+cells& cond=cancer, last accessed March 9, 2019.
Crazzolara R, Kreczy A, Mann G et al. (2001) High expression of the chemokine receptor CXCR4 predicts extramedullary organ infiltration in childhood acute lymphoblastic leukaemia. Br J Haematol 115:545-53.
Davies AM, Sutton BJ (2015) Human IgG4: A structural perspective. Immunological Reviews 268:139-159.
De Falco V, Guarino V, Avilla E et al. (2007) Biological role and potential therapeutic targeting of the chemokine receptor CXCR4 in undifferentiated thyroid cancer. Cancer Res 67:11821-9.
Dewan MZ, Ahmed S, Iwasaki Y et al. (2006) Stromal cell-derived factor-1 and CXCR4 receptor interaction in tumor growth and metastasis of breast cancer. Biomed Pharmacother 60:273-276.
Domanska UM, Kruizinga RC, Nagengast et al. (2013) A review on CXCR4/CXCL12 axis in oncology: No place to hide. Eur J Cancer 49:219-30.
Duda DG, Kozin SV, Kirkpatrick ND et al. (2011) CXCL12 (SDF-1)-CXCR4/CXCR7 pathway inhibition: An emerging sensitizer for anticancer therapies? Clin Cancer Res 17:2074-80.
Farkona S, Diamandis EP, Blasutig IM et al. (2016) Cancer immunotherapy: the beginning of the end of cancer? BMC Medicine 14:73.
Fernández L, Portugal R, Valentin J et al. (2013) In vitro natural killer cell immunotherapy for medulloblastoma. Front Oncol 3(April):94.
Fernández L, Valentin J, Zalacain M (2015) Activated and expanded natural killer cells target osteosarcoma tumor initiating cells in an NKG2D-NKG2DL dependent manner. Cancer Lett 368(1):54-63.
Fujisaki H, Kakuda H, Shimasaki N et al. (2009) Expansion of highly cytotoxic human natural killer cells for cancer cell therapy. Cancer Res 69(9):4010-7.
Gangadhar T, Nandi S, Salgia R (2010) The role of chemokine receptor CXCR4 in lung cancer. Cancer Biol Ther 9:409-16.
Gelmini S, Mangoni M, Castiglione F et al. (2009) The CXCR4/CXCL12 axis in endometrial cancer. Clin Exp Metastasis 26, 261-268.
Ghobrial I, Perez R, Baz R et al. (2014) Phase Ib study of the novel anti-CXCR4 antibody ulocuplumab (BMS-936564) in combination with lenalidomide plus low-dose dexamethasone, or with bortezomib plus dexamethasone in subjects with relapsed or refractory multiple myeloma. 56th American Society of Hematology (ASH) Annual
*Meeting,* San Francisco, December 6-9, 2014, Poster Presentation No. 3483. Guillerey C, Huntington ND, Smyth MJ et al. (2016) Targeting natural killer cells in cancer immunotherapy. Nature Immunol 1025-36.
Han M, Lv S, Zhang Y et al. (2014) The prognosis and clinicopathology of CXCR4 in gastric cancer patients: a meta-analysis. Tumour Biol 35:4589-97.
Hirata H, Hinoda Y, Kikuno N et al. (2007) CXCL12 G801A polymorphism is a risk factor for sporadic prostate cancer susceptibility. Clin Cancer Res 13:5056-62.
Hollinger P, Hudson PJ (2005) Engineered antibody fragments and the rise of single domains. Nature Biotech 23(9):1126-36.
Hsu KC, Keever-Taylor CA, Wilton A et al. (2005) Improved outcome in HLA-identical sibling hematopoietic stem-cell transplantation for acute myelogenous leukemia predicted by KIR and HLA genotypes. Blood 105(12):4878-84.
Idusogie EE, Wong PY, Presta LG et al. (2001) Engineered antibodies with increased activity to recruit complement. J Immunol 166(4):2571-5.
Igarashi T, Wynberg J, Srinivasan R et al. (2004) Enhanced cytotoxicity of allogeneic NK cells with killer immunoglobulin-like receptor ligand incompatibility against melanoma and renal cell carcinoma cells. Blood 104:170-7.
Imai C, Iwamoto S, Campana D (2005) Genetic modification of primary natural killer cells overcomes inhibitory signals and induces specific killing of leukemic cells. Blood 106(1):376-83.
Ishikawa E, Tsuboi K, Saijo K et al. (2004) Autologous natural killer cell therapy for human recurrent malignant glioma. Anticancer Res 24:1861-1871.
Ishikawa T, Nakashiro K, Hara S et al. (2006) CXCR4 expression is associated with lymph-node metastasis of oral squamous cell carcinoma. Int J Oncol 28:61-66.
Ishikawa T, Okayama T, Sakamoto N et al. (2018) Phase I clinical trial of adoptive tr killer cells in combination with IgG1 antibody in patients with gastric or colorectal cancer. Int J Cancer 142: 2599-609.
Jin L, Tabe Y, Konoplev S et al. (2008) CXCR4 up-regulation by imatinib induces chronic myelogenous leukemia (CML) cell migration to bone marrow stroma and promotes survival of quiescent CML cells. Mol Cancer Ther 7:48-58.
Jones J, Marian D, Weich E et al. (2007) CXCR4 chemokine receptor engagement modifies integrin dependent adhesion of renal carcinoma cells. Exp Cell Res 313:4051-65.
Kabat EA, Wu TT, Perry H et al. (1991) Sequences of Proteins of Immunological Interest, Fifth Edition, U.S. Department of Health and Human Services, NIH Publication No. 91-3242.
Kajiyama H, Shibata K, Terauchi M et al. (2008) Involvement of SDF-1alpha/CXCR4 axis in the enhanced peritoneal metastasis of epithelial ovarian carcinoma. Int J Cancer 122:91-99.
Kalinkovich A, Tavor S, Avigdor A et al. (2006) Functional CXCR4-expressing microparticles and SDF-1 correlate with circulating acute myelogenous leukemia cells. Cancer Res 66:11013-20.
Kamiya T, Chang Y-H, Campana D (2016) Expanded and activated natural killer cells for immunotherapy of hepatocellular carcinoma. Cancer Immunol Res 4(7):547-81.
Kamta J, Chaar M, Ande A (2017) Advancing cancer therapy with present and emerging immuno-oncology approaches. Front Oncol 18(7):64.
Krook MA, Nicholls LA, Scannell CA et al. (2014) Stress-induced CXCR4 promotes migration and invasion of ewing sarcoma. Mol Cancer Res 12:953-64.
Kuhne MR, Mulvey T, Belanger B et al. (2013) BMS-936564/MDX-1338: a fully human anti-CXCR4 antibody induces apoptosis in vitro and shows antitumor activity in vivo in hematologic malignancies. Clin Cancer Res 19: 357-66.
Lanier LL (2005) NK cell recognition. Annu Rev Immunol 23:225-74.
Lanier LL, Buck DW, Rhodes L et al. (1988) Interleukin 2 activation of natural killer cells rapidly induces the expression and phosphorylation of the Leu-23 activation antigen. J Exp Med 167:1572-85.
Laverdiere C, Hoang BH, Yang R et al. (2005) Messenger RNA expression levels of CXCR4 correlate with metastatic behavior and outcome in patients with osteosarcoma. Clin Cancer Res 11(7):2561-7.
Leavey PJ, Mascarenhas L, Marina N et al. (2008) Prognostic factors for patients with Ewing sarcoma (EWS) at first recurrence following multi-modality therapy: A report from the Children's Oncology Group. Pediatr Blood Cancer 51(3):334-8.
Lee B, Sharron, M, Montaner LJ et al. (1999) Quantification of CD4, CCR5, and CXCR4 levels on lymphocyte subsets, dendritic cells, and differentially conditioned monocyte-derived macrophages. Proc Natl Acad Sci USA 96:5215-20.
Leivas A, Perez-Martinez A, Blanchard MJ et al. (2016) Novel treatment strategy with autologous activated and expanded natural killer cells plus anti-myeloma drugs for multiple myeloma. Oncoimmunology 5(12):e1250051.
Lesokhin AM, Callahan MK, Postow MA, Wolchok JD (2015) On being less tolerant: enhanced cancer immunosurveillance enabled by targeting checkpoints and agonists of T cell activation. Sci Transl Med 7(280):280sr1.
Li Y-J, Dai Y-L, Zhang WB et al. (2015) Clinicopathological and prognostic significance of chemokine receptor CXCR4 in patients with bone and soft tissue sarcoma: a meta-analysis. Clin Exp Med 9:11101-12.
Libura J, Drukala J, Majka M et al. (2002) CXCR4-SDF-1 signaling is active in rhabdomyosarcoma cells and regulates locomotion, chemotaxis, and adhesion. Blood 100:2597-606.
London L, Perussia B, Trinchieri G (1986) Induction of proliferation in vitro of resting human natural killer cells: IL 2 induces into cell cycle most peripheral blood NK cells, but only a minor subset of low density T cells. J Immunol 137:3845-54.
Long EO, Kim HS, Liu D et al. (2013) Controlling natural killer cell responses: integration of signals for activation and inhibition. Annu Rev Immunol 31:227-58.
Lowry LE, Zehring WA (2017) Potentiation of natural killer cells for cancer immunotherapy: a review of literature. Front Immunol 8:1061.
Lv S, Yang Y, Kwon S et al. (2014) The association of CXCR4 expression with prognosis and clinicopathological indicators in colorectal carcinoma patients: a meta-analysis. Histopathol 64:701-712.
Miki J, Furusato B, Li H et al. (2007) Identification of putative stem cell markers, CD133 and CXCR4, in hTERT-immortalized primary nonmalignant and malignant tumor-derived human prostate epithelial cell lines and in prostate cancer specimens. Cancer Res 67:3153-61.
Miller JS, Soignier Y, Panoskaltsis-Mortari A et al. (2005) Successful adoptive transfer and in vivo expansion of human haploidentical NK cells in patients with cancer. Blood 105(8):3051-7.
Mohle R, Failenschmid C, Bautz F et al. (1999) Overexpression of the chemokine receptor CXCR4 in B cell chronic lymphocytic leukemia is associated with increased functional response to stromal cell-derived factor-1 (SDF-1). Leukemia 13:1954-9.
Moretta L, Locatelli F, Pende D et al. (2011) Killer Ig-like receptor-mediated control of natural killer cell alloreactivity in haploidentical hematopoietic stem cell transplantation. Blood 117(3):764-71.
Nausch N, Cerwenka A (2008) NKG2D ligands in tumor immunity. Oncogene 27(45):5944-58.
Nguyen DX, Bos PD, Massagué J (2009) Metastasis: From dissemination to organ-specific colonization. Nat Rev Cancer 9:274-84.
Olafsen T, Wu AM (2010) Antibody vectors for imaging. Semin NuclMed40(3):167-81.
Onoue T, Uchida D, Begum NM et al. (2006) Epithelial-mesenchymal transition induced by the stromal cell-derived factor-1/CXCR4 system in oral squamous cell carcinoma cells. Int J Oncol 29:1133-8.
Pan J, Mestas J, Burdick MD et al. (2006) Stromal derived factor-1 (SDF-1/CXCL12) and CXCR4 in renal cell carcinoma metastasis. Mol Cancer 5:56.
Passaro D, Irigoyen M, Catherinet C et al. (2015) CXCR4 Is Required for Leukemia-Initiating Cell Activity in T Cell Acute Lymphoblastic Leukemia. Cancer Cell 27(6):769-79.
PCT Publication No. WO 2008/060367, published May 22, 2008 by Medarex, Inc.
PCT Publication No. WO 2013/071068, published May 16, 2013 by Bristol-Myers Squibb.
PCT Publication No. WO 2015069874, published May 14, 2015 by Bristol-Myers Squibb and Dana-Farber Cancer Institute.
PCT Publication No. WO 2016/201425, published December 15, 2016 by Bristol-Myers Squibb.
Pegram HJ, Andrews DM, Smyth MJ et al. (2011) Activating and inhibitory receptors of natural killer cells. Immunol Cell Biol 89(2):216-24.
Pende D, Marcenaro S, Falco M et al. (2009) Anti-leukemia activity of alloreactive NK cells in KIR ligand-mismatched haploidentical HSCT for pediatric patients: evaluation of the functional role of activating KIR and redefinition of inhibitory KIR specificity. Blood 113(13):3119-29.
Pérez-Martínez A, de Prada Vicente I, Fernández L et al. (2012) Natural killer cells can exert a graft-vs-tumor effect in haploidentical stem cell transplantation for pediatric solid tumors. Exp Hematol 40(11):882-91.
Pérez-Martínez A, Fernández L, Valentin J et al. (2015a) A phase I/II trial of interleukin-15-stimulated natural killer cell infusion after haploidentical stem cell transplantation for pediatric refractory solid tumors. Cytotherapy 17(11):1594-603.
Pérez-Martínez A, Leung W, Muñoz E et al. (2009) KIR-HLA receptor-ligand mismatch associated with a graft-versus-tumor effect in haploidentical stem cell transplantation for pediatric metastatic solid tumors. Pediatr Blood Cancer 53(1): 120-4.
Pérez-Martínez A, Valentin J, Fernández L et al. (2015b) Arabinoxylan rice bran (MGN-3/Biobran) enhances natural killer cell-mediated cytotoxicity against neuroblastoma in vitro and in vivo. Cytotherapy 17(5):601-12.
Pitt LA, Tikhonova AN, Hu H et al. (2015) CXCL12-Producing Vascular Endothelial Niches Control Acute T Cell Leukemia Maintenance. Cancer Cell 27(6):755-68.
Rabinowich H, Sedlmayr P, Herberman RB, Whiteside TL (1991) Increased proliferation, lytic activity, and purity of human natural killer cells cocultured with mitogen-activated feeder cells. Cell Immunol 135:454-70.
Rubnitz JE, Inaba H, Ribeiro RC, Pounds S et al. (2010) NKAML: a pilot study to determine the safety and feasibility of haploidentical natural killer cell transplantation in childhood acute myeloid leukemia. J Clin Oncol 28(6):955-9.
Scala S, Ottaiano A, Ascierto PA et al. (2005) Expression of CXCR4 predicts poor prognosis in patients with malignant melanoma. Clin Cancer Res 11:1835-41.
Schimanski CC, Bahre R, Gockel I et al. (2006) Dissemination of hepatocellular carcinoma is mediated via chemokine receptor CXCR4. Br J Cancer 95:210-7.
Seyfried TN, Huysentruyt LC (2013) On the origin of cancer metastasis. Crit Rev Oncog 18(1-2):43-73.
Shi J et al. (2013) NIH Public Access 143:641-653.
Somanchi SS, Lee DA (2016) Ex vivo expansion of human NK cells using K562 engineered to express membrane bound IL21. Methods Mol Biol 1441:175-93.
Stiller CA, Trama A, Serraino D et al. (2013) Descriptive epidemiology of sarcomas in Europe: Report from the RARECARE project. Eur J Cancer 49:684-695.
Szmania S, Lapteva N, Garg T et al. (2015) Fresh ex vivo expanded natural killer cells demonstrate robust proliferation in vivo in high-risk relapsed multiple myeloma (MM) patients. J Immunother 38(1):24-36.
Tavor S, Petit I, Porozov S et al. (2004) CXCR4 regulates migration and development of human acute myelogenous leukemia stem cells in transplanted NOD/SCID mice. Cancer Res 64:2817-24.
U.S. Patent No. 7,767,429, issued August 3, 2010 to Bookbinder et al.
Vela M, Aris M, Llorente M et al. (2015) Chemokine receptor-specific antibodies in cancer immunotherapy: Achievements and challenges. Front Immunol 6:12.
Vela M, Corral D, Carrasco P et al. (2018) Haploidentical IL-15/41BBL activated and expanded natural killer cell infusion therapy after salvage chemotherapy in children with relapsed and refractory leukemia. Cancer Lett 422:107-17.
Verhoeven DHJ, de Hooge ASK, Mooiman ECK et al. (2008) NK cells recognize and lyse Ewing sarcoma cells through NKG2D and DNAM-1 receptor dependent pathways. Mol Immunol 45(15):3917-25.
Vivier E, Raulet DH, Moretta A et al. (2011) Innate or adaptive immunity? The example of natural killer cells. Science 331(6013):44-9.
Wu J, Wu X, Liang W et al. (2014) Clinicopathological and prognostic significance of chemokine receptor CXCR4 overexpression in patients with esophageal cancer: a meta-analysis. Tumour Biol 35:3709-15.
Zheng Y, Dou Y, Duan L et al. (2015) Using chemo-drugs or irradiation to break immune tolerance and facilitate immunotherapy in solid cancer. Cell Immunol 294:54-59.

## Claims

1. A combination of therapeutically effective amounts of:
(a) an isolated population of natural killer (NK) cells; and
(b) an isolated antibody or an antigen-binding portion thereof that binds specifically to C-X-C Chemokine Receptor 4 (CXCR4) expressed on the surface of a cancer cell
for use in a method for treating a subject afflicted with a cancer comprising administering to the subject the combination of the NK cells and the isolated antibody or antigen-binding portion thereof.

2. A therapeutically effective amount of an isolated population of natural killer (NK) cells for use in a method for treating a subject afflicted with a cancer in combination with an isolated antibody or an antigen-binding portion thereof that binds specifically to C-X-C Chemokine Receptor 4 (CXCR4) expressed on the surface of a cancer cell, the method comprising administering to the subject the combination of the NK cells and the isolated antibody or antigen-binding portion thereof.

3. A therapeutically effective amount of an isolated antibody or an antigen-binding portion thereof that binds specifically to C-X-C Chemokine Receptor 4 (CXCR4) expressed on the surface of a cancer cell for use in a method for treating a subject afflicted with a cancer in combination with an isolated population of natural killer (NK) cells, the method comprising administering to the subject the combination of the NK cells and the isolated antibody or antigen-binding portion thereof.

4. The combination, isolated population of NK cells, or isolated antibody or an antigen-binding portion thereof for the use of any one of claims 1-3, wherein the population of NK cells comprises activated and expanded NK (NKAE) cells.

5. The combination, isolated population of NK cells, or isolated antibody or an antigen-binding portion thereof for the use of claim 4, wherein the NKAE cells are produced by stimulating NK cells with IL-2, IL-12, IL-15, and/or IL-21 in combination with feeder cells.

6. The combination, isolated population of NK cells, or isolated antibody or an antigen-binding portion thereof for the use of claim 4 or 5, wherein the NKAE cells are produced by coculturing peripheral blood mononuclear cells from healthy donors with (i) irradiated β-lymphoblastoid cells modified to express a membrane-bound form of interleukin-15 (IL-15) and 41BB ligand, and (ii) interleukin-2 (IL-2).

7. The combination, isolated population of NK cells, or isolated antibody or an antigen-binding portion thereof for the use of claim 6, wherein the modified β-lymphoblastoid cells are K562-mb15-41BBL cells.

8. The combination, isolated population of NK cells, or isolated antibody or an antigen-binding portion thereof for the use of any one of claims 1-7, wherein the NK cells are administered to the subject by intravenous, intraarterial, intraperitoneal or intrathecal injection, or injection into a tumor resection cavity.

9. The combination, isolated population of NK cells, or isolated antibody or an antigen-binding portion thereof for the use of any one of claims 1-8, wherein a dose ranging from about 10⁶ to about 10¹⁴ of said NK cells are administered to the subject weekly.

10. The combination, isolated population of NK cells, or isolated antibody or an antigen-binding portion thereof for the use of any one of claims 1-7, wherein the antibody or antigen-binding portion thereof is a monoclonal antibody or an antigen-binding portion thereof.

11. The combination, isolated population of NK cells, or isolated antibody or an antigen-binding portion thereof for the use of any one of claims 1-10, wherein the subject is a human and the antibody or fragment thereof binds to a human CXCR4 receptor.

12. The combination, isolated population of NK cells, or isolated antibody or an antigen-binding portion thereof for the use of any one of claims 1-11, wherein the antibody or an antigen-binding portion thereof disrupts the interaction between CXCR4 and C-X-C motif chemokine 12 (CXCL12) and inhibits CXCR4/CXCL12 signaling.

13. The combination, isolated population of NK cells, or isolated antibody or an antigen-binding portion thereof for the use of claim 12, wherein the antibody or an antigen-binding portion thereof exhibits one or more of the following characteristics:
(a) binds to CXCR4 on a surface of a cancer cell with a K_{D} of about 1 x 10⁻⁸ M or lower as determined by surface plasmon resonance (SPR);
(b) inhibits binding of CXCL12 to CXCR4 with an EC₅₀ of less than about 30 nM;
(c) inhibits CXCL12-induced calcium flux in cells expressing CXCR4 with an EC₅₀ of less than about 1 nM;
(d) inhibits CXCL12-induced migration of cells expressing CXCR4 with an EC₅₀ of less than about 20 nM;
(e) inhibits capillary tube formation by human umbilical vein endothelial cells;
(f) induces apoptosis in cells expressing CXCR4;
(g) inhibits proliferation of CXCR4⁺ tumor cells *in vitro*;
(h) inhibits CXCR4⁺ tumor cell proliferation and/or induces CXCR4⁺ tumor cell apoptosis *in vivo*;
(i) inhibits metastases of CXCR4⁺ tumor cells; and
(j) increases survival time of a CXCR4⁺ tumor-bearing subject.

14. The combination, isolated population of NK cells, or isolated antibody or an antigen-binding portion thereof for the use of any one of claims 1-13, wherein the anti-CXCR4 antibody or portion thereof comprises the CDR1, CDR2 and CDR3 domains in a heavy chain variable region having the amino acid sequence set forth in SEQ ID NO: 25, and the CDR1, CDR2 and CDR3 domains in a light chain variable region having the amino acid sequence set forth in SEQ ID NO: 29.

15. The combination, isolated population of NK cells, or isolated antibody or an antigen-binding portion thereof for the use of any of claims 1-14, wherein the anti-CXCR4 antibody or portion thereof comprises a heavy chain variable region comprising consecutively linked amino acids having the sequence set forth in SEQ ID NO: 25 or 33, and a light chain variable region comprising consecutively linked amino acids having the sequence set forth in SEQ ID NO: 29 or 37.

16. The combination, isolated population of NK cells, or isolated antibody or an antigen-binding portion thereof for the use of any of claims 1-13, wherein the anti-CXCR4 antibody or portion thereof comprises a heavy chain variable region CDR1 comprising consecutively linked amino acids having the sequence set forth in SEQ ID NO: 1, a heavy chain variable region CDR2 comprising consecutively linked amino acids having the sequence set forth in SEQ ID NO: 5, a heavy chain variable region CDR3 comprising consecutively linked amino acids having the sequence set forth in SEQ ID NO: 9, a light chain variable region CDR1 comprising consecutively linked amino acids having the sequence set forth in SEQ ID NO: 13, a light chain variable region CDR2 comprising consecutively linked amino acids having the sequence set forth in SEQ ID NO: 17, and a light chain variable region CDR3 comprising consecutively linked amino acids having the sequence set forth in SEQ ID NO: 21.

17. The combination, isolated population of NK cells, or isolated antibody or an antigen-binding portion thereof for the use of any one of claims 1-16, wherein the antibody or antigen-binding portion thereof:
(a) cross-competes with ulocuplumab for binding to human CXCR4;
(b) binds to substantially the same epitope in human CXCR4 as does ulocuplumab;
(c) is a chimeric, humanized or human monoclonal antibody or a portion thereof;
(d) comprises a heavy chain constant region which is of a human IgG1, IgG2, or IgG4 isotype;
(e) is ulocuplumab or an antigen-binding portion thereof; and/or
(f) is a human IgG1 variant of ulocuplumab or an antigen-binding portion thereof.

18. The combination, isolated population of NK cells, or isolated antibody or an antigen-binding portion thereof for the use of any one of claims 1-17, wherein the cancer is a solid tumor, optionally wherein the solid tumor is a pediatric tumor, optionally wherein the pediatric tumor is a rhabdomyosarcoma, osteosarcoma, neuroblastoma, retinoblastoma, or Ewing sarcoma.

19. The combination, isolated population of NK cells, or isolated antibody or an antigen-binding portion thereof for the use of claim 18, wherein the solid tumor is a cancer selected from small cell lung cancer (SCLC), non-small cell lung cancer (NSCLC), squamous NSCLC, non-squamous NSCLC, squamous cell cancer, non-small cell lung cancer (NSCLC), pancreatic cancer, pancreatic ductal adenocarcinoma (PDAC), ovarian cancer, cervical cancer, carcinoma of the fallopian tubes, uterine (endometrial) cancer, carcinoma of the endometrium, uterine sarcoma, carcinoma of the cervix, carcinoma of the vagina, carcinoma of the vulva, cancer of the urethra, cancer of the ureter, prostate cancer, metastatic castration-resistant prostate cancer (mCRPC), testicular cancer, penile cancer, bladder cancer, breast cancer, triple negative breast cancer (TNBC), male breast cancer, germ cell tumor, sarcoma, skin cancer, basal cell carcinoma, squamous cell carcinoma, Merkel cell carcinoma, bone cancer, melanoma, head and neck cancer, squamous cell carcinoma of the head and neck (SCCHN), thyroid cancer, oral cancer, mouth cancer, salivary gland cancer, throat cancer, esophageal cancer, gastrointestinal cancer, gastric cancer, cancer of the small intestine, gallbladder and bile duct cancer, colorectal cancer, colon carcinoma, rectal cancer, anal cancer, liver cancer, hepatoma, kidney cancer, renal cell carcinoma, cancer of the endocrine system, tumors of the thymus gland, thymona, cancer of the parathyroid gland, cancer of the adrenal gland, soft tissue sarcoma, mesothelioma, carcinoma of the renal pelvis, neoplasm of the central nervous system (CNS), primary CNS lymphoma, tumor angiogenesis, spinal axis tumor, brain cancer, glioma, brain stem glioma, glioblastoma, glioblastoma multiforme (GBM), neuroblastoma, pituitary adenoma, epidermoid cancer, solid tumors of childhood, pediatric sarcoma, metastatic cancer, cancer of unknown primary origin, environmentally-induced cancers, virus-related cancers, AIDS-related cancers, Kaposi's sarcoma, cancers of viral origin, advanced, refractory and/or recurrent solid tumors, and any combination of the preceding solid tumors.

20. The combination, isolated population of NK cells, or isolated antibody or an antigen-binding portion thereof for the use of any one of claims 1-17, wherein the cancer is a hematological malignancy.

21. The combination, isolated population of NK cells, or isolated antibody or an antigen-binding portion thereof for the use of claim 20, wherein the hematological malignancy is selected from acute lymphoblastic leukemia (ALL), acute myelogenous leukemia (AML), chronic lymphocytic leukemia (CLL), chronic myelogenous leukemia (CML), Hodgkin's lymphoma (HL), non-Hodgkin's lymphomas (NHLs), Burkitt's lymphoma, diffuse large B-cell lymphoma (DLBCL), follicular lymphoma (FL), immunoblastic large cell lymphoma, precursor B-lymphoblastic lymphoma, mantle cell lymphoma, mycosis fungoides, anaplastic large cell lymphoma, precursor T-lymphoblastic lymphoma, sinonasal natural killer/T-cell lymphoma, multiple myeloma (MM), myelodysplastic syndrome (MDS), smoldering myeloma, monoclonal gammopathy of undetermined significance (MGUS), advanced, metastatic, refractory and/or recurrent hematological malignancies, and any combination of the preceding hematological malignancies.

22. The combination, isolated population of NK cells, or isolated antibody or an antigen-binding portion thereof for the use of any one of claims 1-21, wherein the antibody or antigen-binding portion thereof is administered:
(a) at a flat dose of about 50 to about 2000 mg once or twice about every week, once about every 2 weeks, or once about every 3 weeks;
(b) at a flat dose of about 200, about 400, about 800, about 1600, or about 2000 mg once about every week or once about every 2 weeks; and/or
(c) to the subject by intravenous or subcutaneous administration.

23. The combination, isolated population of NK cells, or isolated antibody or an antigen-binding portion thereof for the use of any one of claims 1-22, wherein:
(a) the NK cells and the antibody or antigen-binding portion thereof are administered
sequentially to the subject;
(b) the NK cells are administered before the antibody or antigen-binding portion thereof;
(c) the antibody or antigen-binding portion thereof is administered before the NK cells;
(d) the NK cells and the antibody or antigen-binding portion thereof are administered concurrently in separate compositions; or
(e) the NK cells and the antibody or antigen-binding portion thereof are admixed in a single composition and administered concurrently.

24. A kit for use in treating a subject afflicted with a cancer, the kit comprising:
(a) one or more dosages ranging from about 50 to about 2000 mg of an antibody or an antigen-binding portion thereof that binds specifically to CXCR4 expressed on the surface of a cancer cell;
(b) one or more dosages ranging from about 10⁶ to about 10¹⁴ of a population of NKAE cells; and
(c) instructions for using the antibody or portion thereof and the NKAE cells in the use of any one of claims 2-23.

## Patentansprüche

1. Kombination therapeutisch wirksamer Mengen von:
(a) einer isolierten Population natürlicher Killer-(NK-)Zellen; und
(b) einem isolierten Antikörper oder einem antigenbindenden Abschnitt davon, der spezifisch an C-X-C-Motiv-Chemokinrezeptor 4 (CXCR4) bindet, der auf der Oberfläche einer Krebszelle exprimiert wird,
zur Verwendung in einem Verfahren zur Behandlung von einem an einem Krebs leidenden Individuum, welches Verabreichen, an das Individuum, der Kombination von den NK-Zellen und dem isolierten Antikörper oder antigenbindenden Abschnitt davon umfasst.

2. Therapeutisch wirksame Menge von einer isolierten Population natürlicher Killer-(NK-)Zellen zur Verwendung in einem Verfahren zur Behandlung von einem an einem Krebs leidenden Individuum in Kombination mit einem isolierten Antikörper oder einem antigenbindenden Abschnitt davon, der spezifisch an C-X-C-Motiv-Chemokinrezeptor 4 (CXCR4) bindet, der auf der Oberfläche einer Krebszelle exprimiert wird, wobei das Verfahren Verabreichen, an das Individuum, der Kombination von den NK-Zellen und dem isolierten Antikörper oder antigenbindenden Abschnitt davon umfasst.

3. Therapeutisch wirksame Menge von einem isolierten Antikörper oder einem antigenbindenden Abschnitt davon, der spezifisch an C-X-C-Motiv-Chemokinrezeptor 4 (CXCR4) bindet, der auf der Oberfläche einer Krebszelle exprimiert wird, zur Verwendung in einem Verfahren zur Behandlung von einem an einem Krebs leidenden Individuum in Kombination mit einer isolierten Population natürlicher Killer-(NK-)Zellen, wobei das Verfahren Verabreichen, an das Individuum, der Kombination von den NK-Zellen und dem isolierten Antikörper oder antigenbindenden Abschnitt davon umfasst.

4. Kombination, isolierte Population von NK-Zellen, oder isolierter Antikörper oder antigenbindender Abschnitt davon zur Verwendung nach einem der Ansprüche 1 bis 3, wobei die Population von NK-Zellen aktivierte und expandierte NK-(NKAE-)Zellen umfasst.

5. Kombination, isolierte Population von NK-Zellen, oder isolierter Antikörper oder antigenbindender Abschnitt davon zur Verwendung nach Anspruch 4, wobei die NKAE-Zellen durch Stimulieren von NK-Zellen mit IL-2, IL-12, IL-15 und/oder IL-21 in Kombination mit Fütterzellen produziert werden.

6. Kombination, isolierte Population von NK-Zellen, oder isolierter Antikörper oder antigenbindender Abschnitt davon zur Verwendung nach Anspruch 4 oder 5, wobei die NKAE-Zellen produziert werden durch Ko-Kultivieren mononukleärer Zellen aus peripherem Blut von gesunden Spendern mit (i) bestrahlten β-Lymphoblastoidzellen, modifiziert zwecks Expression von einer membrangebundenen Form von Interleukin-15 (II,-15) und einem 41BB Ligand, und (ii) Interleukin-2 (IL-2).

7. Kombination, isolierte Population von NK-Zellen, oder isolierter Antikörper oder antigenbindender Abschnitt davon zur Verwendung nach Anspruch 6, wobei die modifizierten β-Lymphoblastoidzellen K562-mb15-41BBL-Zellen sind.

8. Kombination, isolierte Population von NK-Zellen, oder isolierter Antikörper oder antigenbindender Abschnitt davon zur Verwendung nach einem der Ansprüche 1 bis 7, wobei die NK-Zellen dem Individuum durch intravenöse, intraarterielle, intraperitoneale oder intrathekale Injektion, oder durch Injektion in eine Tumorresektionskavität, verabreicht werden.

9. Kombination, isolierte Population von NK-Zellen, oder isolierter Antikörper oder antigenbindender Abschnitt davon zur Verwendung nach einem der Ansprüche 1 bis 8, wobei dem Individuum wöchentlich eine Dosis im Bereich von etwa 10⁶ bis etwa 10¹⁴ der NK-Zellen verabreicht wird.

10. Kombination, isolierte Population von NK-Zellen, oder isolierter Antikörper oder antigenbindender Abschnitt davon zur Verwendung nach einem der Ansprüche 1 bis 7, wobei der Antikörper oder der antigenbindende Abschnitt davon ein monoklonaler Antikörper oder ein antigenbindender Abschnitt davon ist.

11. Kombination, isolierte Population von NK-Zellen, oder isolierter Antikörper oder antigenbindender Abschnitt davon zur Verwendung nach einem der Ansprüche 1 bis 10, wobei das Individuum ein Mensch ist und der Antikörper oder das Fragment davon an einen humanen CXCR4-Rezeptor bindet.

12. Kombination, isolierte Population von NK-Zellen, oder isolierter Antikörper oder antigenbindender Abschnitt davon zur Verwendung nach einem der Ansprüche 1 bis 11, wobei der Antikörper oder antigenbindende Abschnitt davon die Wechselwirkung zwischen CXCR4 und CXC-Motiv-Chemokin 12 (CXCL12) stört und CXCR4/CXCL12-Signalgebung hemmt.

13. Kombination, isolierte Population von NK-Zellen, oder isolierter Antikörper oder antigenbindender Abschnitt davon zur Verwendung nach Anspruch 12, wobei der Antikörper oder ein antigenbindender Abschnitt davon eine oder mehrere der folgenden Charakteristiken aufweist:
(a) bindet sich an CXCR4 auf einer Oberfläche einer Krebszelle mit einer K_{D} von etwa 1 x 10⁻⁸M oder weniger gemäß Bestimmung durch Oberflächenplasmonenresonanz (SPR);
(b) hemmt Bindung von CXCL12 an CXCR4 mit einer EC₅₀ von weniger als etwa 30 nM;
(c) hemmt einen CXCL12-induzierten Kalziumflux in CXCR4 exprimierenden Zellen mit einer EC₅₀ von weniger als etwa 1 nM;
(d) hemmt CXCL12-induzierte Migration von CXCR4 exprimierenden Zellen mit einer EC₅₀ von weniger als etwa 20 nM;
(e) hemmt Kapillarenbildung durch endotheliale Zellen der humanen Nabelvene;
(f) induziert Apoptose in CXCR4 exprimierenden Zellen;
(g) hemmt Proliferation von CXCR4⁺-Tumorzellen *in vitro*;
(h) hemmt CXCR4⁺-Tumorzellproliferation und/oder induziert CXCR4⁺-Tumorzellapoptose *in vivo*;
(i) hemmt Metastasen von CXCR4⁺-Tumorzellen; und
(j) steigert die Überlebenszeit eines Individuums, das einen CXCR4⁺-Tumor trägt.

14. Kombination, isolierte Population von NK-Zellen, oder isolierter Antikörper oder antigenbindender Abschnitt davon zur Verwendung nach einem der Ansprüche 1 bis 13, wobei der Anti-CXCR4-Antikörper oder Abschnitt davon die Domänen CDR1, CDR2 und CDR3 in einer variablen Schwerkettenregion mit der in SEQ ID NO: 25 aufgeführten Aminosäuresequenz und die Domänen CDR1, CDR2 und CDR3 in einer variablen Leichtkettenregion mit der in SEQ ID NO: 29 aufgeführten Aminosäuresequenz umfasst.

15. Kombination, isolierte Population von NK-Zellen, oder isolierter Antikörper oder antigenbindender Abschnitt davon zur Verwendung nach einem der Ansprüche 1 bis 14, wobei der Anti-CXCR4-Antikörper oder Abschnitt davon eine variable Schwerkettenregion, umfassend fortlaufend verknüpfte Aminosäuren mit der in SEQ ID NO: 25 oder 33 aufgeführten Sequenz, und eine variable Leichtkettenregion, umfassend fortlaufend verknüpfte Aminosäuren mit der in SEQ ID NO: 29 oder 37 aufgeführten Sequenz, umfasst.

16. Kombination, isolierte Population von NK-Zellen, oder isolierter Antikörper oder antigenbindender Abschnitt davon zur Verwendung nach einem der Ansprüche 1 bis 13, wobei der Anti-CXCR4-Antikörper oder Abschnitt davon eine variable Schwerkettenregion CDR1, umfassend fortlaufend verknüpfte Aminosäuren mit der in SEQ ID NO: 1 aufgeführten Sequenz, eine variable Schwerkettenregion CDR2, umfassend fortlaufend verknüpfte Aminosäuren mit der in SEQ ID NO: 5 aufgeführten Sequenz, eine variable Schwerkettenregion CDR3, umfassend fortlaufend verknüpfte Aminosäuren mit der in SEQ ID NO: 9 aufgeführten Sequenz, eine variable Leichtkettenregion CDR1, umfassend fortlaufend verknüpfte Aminosäuren mit der in SEQ ID NO: 13 aufgeführten Sequenz, eine variable Leichtkettenregion CDR2, umfassend fortlaufend verknüpfte Aminosäuren mit der in SEQ ID NO: 17 aufgeführten Sequenz, und eine variable Leichtkettenregion CDR3, umfassend fortlaufend verknüpfte Aminosäuren mit der in SEQ ID NO: 21 aufgeführten Sequenz, umfasst.

17. Kombination, isolierte Population von NK-Zellen, oder isolierter Antikörper oder antigenbindender Abschnitt davon zur Verwendung nach einem der Ansprüche 1 bis 16, wobei der Antikörper oder antigenbindende Abschnitt davon:
(a) mit Ulocuplumab um Bindung an humanes CXCR4 kreuzkonkurriert;
(b) an im Wesentlichen das gleiche Epitop in humanem CXCR4 wie Ulocuplumab bindet;
(c) ein chimärer, humanisierter oder humaner monoklonaler Antikörper oder ein Abschnitt davon ist;
(d) eine konstante Schwerkettenregion umfasst, die von einem humanen IgG1-, IgG2- oder IgG4-Isotyp ist;
(e) Ulocuplumab oder ein antigenbindender Abschnitt davon ist; und/oder
(f) eine Human-IgG1-Variante von Ulocuplumab oder ein antigenbindender Abschnitt davon ist.

18. Kombination, isolierte Population von NK-Zellen, oder isolierter Antikörper oder antigenbindender Abschnitt davon zur Verwendung nach einem der Ansprüche 1 bis 17, wobei der Krebs ein solider Tumor ist, wobei, wahlweise, der solide Tumor ein pädiatrischer Tumor ist, wobei, wahlweise, der pädiatrische Tumor ein Rhabdomyosarkom, Osteosarkom, Neuroblastom, Retinoblastom oder Ewing-Sarkom ist.

19. Kombination, isolierte Population von NK-Zellen, oder isolierter Antikörper oder antigenbindender Abschnitt davon zur Verwendung nach Anspruch 18, wobei der solide Tumor ein Krebs ist, der ausgewählt ist aus kleinzelligem Lungenkrebs (SCLC), nichtkleinzelligem Lungenkrebs (NSCLC), squamösem NSCLC, nichtsquamösem NSCLC, Plattenepithelkrebs, nichtkleinzelligem Lungenkrebs (NSCLC), Pankreaskrebs, duktalem Adenokarzinom des Pankreas (PDAC), Eierstockkrebs, Zervixkrebs, Karzinom der Eilleiter, Gebärmutter-(Endometrium-)Krebs, Karzinom des Endometriums, Gebärmuttersarkom, Karzinom der Zervix, Karzinom der Vagina, Karzinom der Vulva, Krebs der Harnröhre, Krebs des Harnleiters, Prostatakrebs, metastatischem kastrationsresistentem Prostatakrebs (mCRPC), Hodenkrebs, Peniskrebs, Blasenkrebs, Brustkrebs, triple-negativem Brustkrebs (TNBC), Brustkrebs beim Mann, Keimzelltumor, Sarkom, Hautkrebs, Basalzellkarzinom, Plattenepithelkarzinom, Merkelzellkarzinom, Knochenkrebs, Melanom, Kopf-Hals-Krebs, Plattenepithelkarzinom des Kopfes und des Halses (SCCHN), Schilddrüsenkrebs, oralem Krebs, Mundkrebs, Speicheldrüsenkrebs, Halskrebs, Speiseröhrenkrebs, gastrointestinalem Krebs, Magenkrebs, Dünndarmkrebs, Gallenblasen- und Gallengangkrebs, kolorektalem Krebs, Kolonkarzinom, rektalem Krebs, analem Krebs, Leberkrebs, Hepatom, Nierenkrebs, Nierenzellkarzinom, Krebs des endokrinen Systems, Tumoren der Thymusdrüse, Thymom, Krebs der Nebenschilddrüse, Krebs der Nebenniere, Weichteilsarkom, Mesotheliom, Karzinom des Nierenbeckens, Neoplasma des Zentralnervensystems (ZNS), primärem ZNS-Lymphom, Tumorangiogenese, Rückenmarkstumor, Hirnkrebs, Gliom, Hirnstammgliom, Glioblastom, Glioblastoma multiforme (GBM), Neuroblastom, Hypophysenadenom, Epidermoidkrebs, soliden Tumoren des Kindes, pädiatrischem Sarkom, metastatischem Krebs, Krebs unbekannten primären Ursprungs, umweltinduzierten Krebsen, virusbedingten Krebsen, AIDS-bedingten Krebsen, Kaposi-Sarkom, Krebsen viralen Ursprungs, fortgeschrittenen, refraktären und/oder rezidivierenden soliden Tumoren, sowie jeder beliebigen Kombination der vorstehenden soliden Tumoren.

20. Kombination, isolierte Population von NK-Zellen, oder isolierter Antikörper oder antigenbindender Abschnitt davon zur Verwendung nach einem der Ansprüche 1 bis 17, wobei der Krebs eine maligne hämatologische Erkrankung ist.

21. Kombination, isolierte Population von NK-Zellen, oder isolierter Antikörper oder antigenbindender Abschnitt davon zur Verwendung nach Anspruch 20, wobei die maligne hämatologische Erkrankung ausgewählt ist aus akuter lymphatischer Leukämie (ALL), akuter myeloischer Leukämie (AML), chronischer lymphatischer Leukämie (CLL), chronischer myeloischer Leukämie (CML), Hodgkin-Lymphom (HL), Non-Hodgkin-Lymphomen (NHLs), Burkitt-Lymphom, diffusem großzelligem B-Zell-Lymphom (DLBCL), follikulärem Lymphom (FL), immunoblastischem großzelligem Lymphom, lymphoblastischem B-Zellen-Vorläufer-Lymphom, Mantelzelllymphom, Mycosis fungoides, anaplastischem großzelligem Lymphom, lymphoblastischem T-Zellen-Vorläufer-Lymphom, sinonasalem Natürliche-Killer-/T-Zellen-Lymphom, multiplem Myelom (MM), myelodysplastischem Syndrom (MDS), schwelendem Myelom, monoklonaler Gammopathie unklarer Signifikanz (MGUS), fortgeschrittenen, metastatischen, refraktären und/oder rezidivierenden malignen hämatologischen Erkrankungen, sowie jeder beliebigen Kombination der vorstehenden malignen hämatologischen Erkrankungen.

22. Kombination, isolierte Population von NK-Zellen, oder isolierter Antikörper oder antigenbindender Abschnitt davon zur Verwendung nach einem der Ansprüche 1 bis 21, wobei der Antikörper oder antigenbindende Abschnitt davon verabreicht wird:
(a) in einer Festdosis von etwa 50 bis etwa 2000 mg einmal oder zweimal etwa jede Woche, einmal etwa alle 2 Wochen, oder einmal etwa alle 3 Wochen;
(b) in einer Festdosis von etwa 200, etwa 400, etwa 800, etwa 1600 oder etwa 2000 mg einmal etwa jede Woche oder einmal etwa alle 2 Wochen; und/oder
(c) dem Individuum durch intravenöse oder subkutane Verabreichung.

23. Kombination, isolierte Population von NK-Zellen, oder isolierter Antikörper oder antigenbindender Abschnitt davon zur Verwendung nach einem der Ansprüche 1 bis 22, wobei:
(a) die NK-Zellen und der Antikörper oder antigenbindende Abschnitt davon dem Individuum in Abfolge verabreicht werden;
(b) die NK-Zellen vor dem Antikörper oder antigenbindenden Abschnitt davon verabreicht werden;
(c) der Antikörper oder antigenbindende Abschnitt davon vor den NK-Zellen verabreicht wird;
(d) die NK-Zellen und der Antikörper oder antigenbindende Abschnitt davon in separaten Zusammensetzungen gleichzeitig verabreicht werden; oder
(e) die NK-Zellen und der Antikörper oder antigenbindende Abschnitt davon in einer einzigen Zusammensetzung vermischt und gleichzeitig verabreicht werden.

24. Kit zur Verwendung bei Behandlung von einem an einem Krebs leidenden Individuum, wobei das Kit umfasst:
(a) eine oder mehrere Dosierungen im Bereich von etwa 50 bis etwa 2000 mg eines Antikörpers oder eines antigenbindenden Abschnitts davon, der spezifisch an CXCR4 bindet, der auf der Oberfläche einer Krebszelle exprimiert wird;
(b) eine oder mehrere Dosierungen im Bereich von etwa 10⁶ bis etwa 10¹⁴ aus einer Population von NKAE-Zellen; und
(c) Anweisungen zur Verwendung des Antikörpers oder Abschnitts davon und der NKAE-Zellen bei der Verwendung nach einem der Ansprüche 2 bis 23.

## Revendications

1. Combinaison de quantités thérapeutiquement efficaces :
(a) d'une population isolée de cellules tueuses naturelles (NK) ; et
(b) d'un anticorps isolé ou d'une partie de liaison à un antigène de celui-ci qui se lie spécifiquement au récepteur des C-X-C chimiokines de type 4 (CXCR4) exprimé sur la surface d'une cellule cancéreuse
pour utilisation dans un procédé de traitement d'un sujet atteint d'un cancer comprenant l'administration au sujet de la combinaison de cellules NK et de l'anticorps isolé ou de la partie de liaison à un antigène de celui-ci.

2. Quantité thérapeutiquement efficace d'une population isolée de cellules tueuses naturelles (NK) pour utilisation dans un procédé de traitement d'un sujet atteint d'un cancer en combinaison avec un anticorps isolé ou une partie de liaison à un antigène de celui-ci qui se lie spécifiquement au récepteur des C-X-C chimiokines de type 4 (CXCR4) exprimé sur la surface d'une cellule cancéreuse, le procédé comprenant l'administration au sujet de la combinaison des cellules NK et de l'anticorps isolé ou de la partie de liaison à un antigène de celui-ci.

3. Quantité thérapeutiquement efficace d'un anticorps isolé ou d'une partie de liaison à un antigène de celui-ci qui se lie spécifiquement au récepteur des C-X-C chimiokines de type 4 (CXCR4) exprimé sur la surface d'une cellule cancéreuse pour utilisation dans un procédé de traitement d'un sujet atteint d'un cancer en combinaison avec une population isolée de cellules tueuses naturelles (NK), le procédé comprenant l'administration au sujet de la combinaison des cellules NK et de l'anticorps isolé ou de la partie de liaison à un antigène de celui-ci.

4. Combinaison, population isolée de cellules NK, ou anticorps isolé ou partie de liaison à un antigène de celui-ci pour utilisation selon l'une quelconque des revendications 1-3, où la population de cellules NK comprend des cellules NK activées et amplifiées (NKAE).

5. Combinaison, population isolée de cellules NK, ou anticorps isolé ou partie de liaison à un antigène de celui-ci pour utilisation selon la revendication 4, où les cellules NKAE sont produites en stimulant des cellules NK avec IL-2, IL-12, IL-15 et/ou IL-21 en combinaison avec des cellules nourricières.

6. Combinaison, population isolée de cellules NK, ou anticorps isolé ou partie de liaison à un antigène de celui-ci pour utilisation selon la revendication 4 ou 5, où les cellules NKAE sont produites en mettant en co-culture des cellules mononucléaires de sang périphérique provenant de donneurs sains avec (i) des cellules β-lymphoblastoïdes irradiées modifiées pour exprimer une forme liée à la membrane d'interleukine-15 (IL-15) et un ligand 41BB, et (ii) de l'interleukine-2 (IL-2).

7. Combinaison, population isolée de cellules NK, ou anticorps isolé ou partie de liaison à un antigène de celui-ci pour utilisation selon la revendication 6, où les cellules β-lymphoblastoïdes modifiées sont des cellules K562-mb15-41BBL.

8. Combinaison, population isolée de cellules NK, ou anticorps isolé ou partie de liaison à un antigène de celui-ci pour utilisation selon l'une quelconque des revendications 1 à 7, où les cellules NK sont administrées au sujet par injection intraveineuse, intraartérielle, intrapéritonéale ou intrathécale, ou par injection dans une cavité de résection tumorale.

9. Combinaison, population isolée de cellules NK, ou anticorps isolé ou partie de liaison à un antigène de celui-ci pour utilisation selon l'une quelconque des revendications 1 à 8, où une dose allant d'environ 10⁶ à environ 10¹⁴ desdites cellules NK est administrée toutes les semaines au sujet.

10. Combinaison, population isolée de cellules NK, ou anticorps isolé ou partie de liaison à un antigène de celui-ci pour utilisation selon l'une quelconque des revendications 1 à 7, où l'anticorps ou la partie de liaison à un antigène de celui-ci est un anticorps monoclonal ou une partie de liaison à un antigène de celui-ci.

11. Combinaison, population isolée de cellules NK, ou anticorps isolé ou partie de liaison à un antigène de celui-ci pour utilisation selon l'une quelconque des revendications 1 à 10, où le sujet est un être humain et l'anticorps ou le fragment de celui-ci se lie à un récepteur CXCR4 humain.

12. Combinaison, population isolée de cellules NK, ou anticorps isolé ou partie de liaison à un antigène de celui-ci pour utilisation selon l'une quelconque des revendications 1 à 11, où l'anticorps ou la partie de liaison à un antigène de celui-ci perturbe l'interaction entre CXCR4 et une chimiokine (motif C-X-C) ligand 12 (CXCL12) et inhibe la signalisation de CXCR4/CXCL12.

13. Combinaison, population isolée de cellules NK, ou anticorps isolé ou partie de liaison à un antigène de celui-ci pour utilisation selon la revendication 12, où l'anticorps ou une partie de liaison à un antigène de celui-ci présente une ou plusieurs des caractéristiques suivantes :
(a) se lie à CXCR4 sur une surface d'une cellule cancéreuse avec une K_{D} d'environ 1 x 10⁻⁸ M ou moins comme déterminée par résonance plasmonique de surface (SPR) ;
(b) inhibe la liaison de CXCL12 à CXCR4 avec une CE₅₀ de moins d'environ 30 nM;
(c) inhibe un flux de calcium induit par CXCL12 dans des cellules exprimant CXCR4 avec une CE₅₀ de moins d'environ 1 nM ;
(d) inhibe la migration de cellules exprimant CXCR4 induite par CXCL12 avec une CE₅₀ de moins d'environ 20 nM ;
(e) inhibe la formation de tubes capillaires par des cellules endothéliales de veine ombilicale humaine ;
(f) induit l'apoptose dans des cellules exprimant CXCR4 ;
(g) inhibe la prolifération de cellules tumorales CXCR4⁺ *in vitro* ;
(h) inhibe la prolifération de cellule tumorales CXCR4⁺ et/ou induit l'apoptose de cellules tumorales CXCR4⁺ *in vivo* ;
(i) inhibe les métastases de cellules tumorales CXCR4⁺ ; et
(j) augmente le temps de survie d'un sujet porteur d'une tumeur CXCR4⁺.

14. Combinaison, population isolée de cellules NK, ou anticorps isolé ou partie de liaison à un antigène de celui-ci pour utilisation selon l'une quelconque des revendications 1 à 13, où l'anticorps anti-CXCR4 ou la partie de celui-ci comprend les domaines CDR1, CDR2 et CDR3 dans une région variable de chaîne lourde ayant la séquence d'acides aminés présentée dans la SEQ ID NO : 25, et les domaines CDR1, CDR2 et CDR3 dans une région variable de chaîne légère ayant la séquence d'acides aminés présentée dans la SEQ ID NO : 29.

15. Combinaison, population isolée de cellules NK, ou anticorps isolé ou partie de liaison à un antigène de celui-ci pour utilisation selon l'une quelconque des revendications 1 à 14, où l'anticorps anti-CXCR4 ou la partie de celui-ci comprend une région variable de chaîne lourde comprenant des acides aminés liés consécutivement ayant la séquence présentée dans la SEQ ID NO : 25 ou 33, et une région variable de chaîne légère comprenant des acides aminés liés consécutivement ayant la séquence présentée dans la SEQ ID NO : 29 ou 37.

16. Combinaison, population isolée de cellules NK, ou anticorps isolé ou partie de liaison à un antigène de celui-ci pour utilisation selon l'une quelconque des revendications 1 à 13, où l'anticorps anti-CXCR4 ou la partie de celui-ci comprend une CDR1 de région variable de chaîne lourde comprenant des acides aminés liés consécutivement ayant la séquence présentée dans la SEQ ID NO : 1, une CDR2 de région variable de chaîne lourde comprenant des acides aminés liés consécutivement ayant la séquence présentée dans la SEQ ID NO : 5, une CDR3 de région variable de chaîne lourde comprenant des acides aminés liés consécutivement ayant la séquence présentée dans la SEQ ID NO : 9, une CDR1 de région variable de chaîne légère comprenant des acides aminés liés consécutivement ayant la séquence présentée dans la SEQ ID NO : 13, une CDR2 de région variable de chaîne légère comprenant des acides aminés liés consécutivement ayant la séquence présentée dans la SEQ ID NO : 17, une CDR3 de région variable de chaîne légère comprenant des acides aminés liés consécutivement ayant la séquence présentée dans la SEQ ID NO : 21.

17. Combinaison, population isolée de cellules NK, ou anticorps isolé ou partie de liaison à un antigène de celui-ci pour utilisation selon l'une quelconque des revendications 1 à 16, où l'anticorps ou la partie de liaison à un antigène de celui-ci :
(a) rivalise avec l'ulocuplumab pour se lier à CXCR4 humain ;
(b) se lie sensiblement au même épitope dans CXCR4 humain que l'ulocuplumab ;
(c) est un anticorps monoclonal chimérique, humanisé ou humain ou une partie de celui-ci ;
(d) comprend une région constante de chaîne lourde qui est un isotype d'IgG1, d'IgG2 ou d'IgG4 humaine ;
(e) est de l'ulocuplumab ou une partie de liaison à un antigène de celui-ci, et/ou
(f) est un variant de l'IgG1 humaine d'ulocuplumab ou d'une partie de liaison à un antigène de celui-ci.

18. Combinaison, population isolée de cellules NK, ou anticorps isolé ou partie de liaison à un antigène de celui-ci pour utilisation selon l'une quelconque des revendications 1 à 17, où le cancer est une tumeur solide, la tumeur solide étant optionnellement une tumeur pédiatrique, la tumeur pédiatrique étant optionnellement un rhabdomyosarcome, ostéosarcome, neuroblastome, rétinoblastome ou un sarcome d'Ewing.

19. Combinaison, population isolée de cellules NK, ou anticorps isolé ou partie de liaison à un antigène de celui-ci pour utilisation selon la revendication 18, où la tumeur solide est un cancer sélectionné parmi un cancer du poumon à petites cellules (CPPC), cancer du poumon non à petites cellules (CPNPC), CPNPC squameux, CPNPC non squameux, cancer à cellules squameuses, cancer du poumon non à petites cellules (CPNPC), cancer du pancréas, adénocarcinome canalaire du pancréas (ACCP), cancer de l'ovaire, cancer du col de l'utérus, carcinome des trompes de Fallope, cancer utérin (de l'endomètre), carcinome de l'endomètre, sarcome utérin, carcinome du col de l'utérus, carcinome du vagin, carcinome de la vulve, cancer de l'urètre, cancer de l'uretère, cancer de la prostate, cancer métastatique de la prostate résistant à la castration (CPRCm), cancer des testicules, cancer du pénis, cancer de la vessie, cancer du sein, cancer du sein triple négatif (CSTN), cancer du sein chez l'homme, tumeur des cellules germinales, sarcome, cancer de la peau, carcinome à cellules basales, carcinome à cellules squameuses, carcinome à cellules de Merkel, cancer des os, mélanome, cancer de la tête et du cou, carcinome épidermoïde de la tête et du cou (CETC), cancer de la thyroïde, cancer buccal, cancer de la bouche, cancer des glandes salivaires, cancer de la gorge, cancer de l'oesophage, cancer gastrointestinal, cancer de l'estomac, cancer de l'intestin grêle, cancer de la vésicule biliaire et du canal biliaire, cancer colorectal, carcinome du côlon, cancer rectal, cancer anal, cancer du foie, hépatome, cancer du rein, carcinome à cellules rénales, cancer du système endocrinien, tumeurs du thymus, thymoma, cancer de la glande parathyroïde, cancer de la glande surrénale, sarcome des tissus mous, mésothéliome, carcinome du bassinet rénal, néoplasme du système nerveux central (SNC), lymphome primaire du SNC, angiogenèse tumorale, tumeur de l'axe rachidien, cancer du cerveau, gliome, gliome du tronc cérébral, glioblastome, glioblastome multiforme (GBM), neuroblastome, adénome hypophysaire, cancer épidermoïde, tumeurs solides de l'enfance, sarcome pédiatrique, cancer métastatique, cancer d'origine primaire inconnue, cancers induits par l'environnement, cancers liés à des virus, cancers liés au SIDA, sarcome de Kaposi, cancers d'origine virale, tumeurs solides avancées, réfractaires et/ou récurrentes et une combinaison quelconque des tumeurs solides précédentes.

20. Combinaison, population isolée de cellules NK, ou anticorps isolé ou partie de liaison à un antigène de celui-ci pour utilisation selon l'une quelconque des revendications 1 à 17, où le cancer est une hémopathie maligne.

21. Combinaison, population isolée de cellules NK, ou anticorps isolé ou partie de liaison à un antigène de celui-ci pour utilisation selon la revendication 20, où l'hémopathie maligne est sélectionnée parmi une leucémie lymphoblastique aiguë (LLA), leucémie myéloïde aiguë (LMA), leucémie lymphocytaire chronique (LLC), leucémie myéloïde chronique (LMC), lymphome de Hodgkin (LH), lymphome non hodgkinien (LNH), lymphome de Burkitt, lymphome diffus à grandes cellules B (LDGCB), lymphome folliculaire (LF), lymphome immunoblastique à grandes cellules, lymphome lymphoblastique à précurseurs B, lymphome à cellules du manteau, mycosis fongoïde, lymphome anaplasique à grandes cellules, lymphome lymphoblastique à précurseurs T, lymphome à cellules T/tueuses naturelles des fosses nasales et des sinus, myélome multiple (MM), syndrome myélodysplasique (SMD), myélome latent, gammopathie monoclonale de signification indéterminée (GMSI), hémopathies malignes métastatiques, réfractaires et/ou récurrentes, et une combinaison quelconque des hémopathies malignes précédentes.

22. Combinaison, population isolée de cellules NK, ou anticorps isolé ou partie de liaison à un antigène de celui-ci pour utilisation selon l'une quelconque des revendications 1 à 21, où l'anticorps ou la partie de liaison à un antigène de celui-ci est administré :
(a) en une dose fixe d'environ 50 à environ 2000 mg une fois ou deux fois environ toutes les semaines, une fois environ toutes les 2 semaines ou une fois environ toutes les 3 semaines ;
(b) en une dose fixe d'environ 200, d'environ 400, d'environ 800, d'environ 1600 ou d'environ 2000 mg une fois environ toutes les semaines ou une fois environ toutes les 2 semaines ; et/ou
(c) au sujet par administration intraveineuse ou sous-cutanée.

23. Combinaison, population isolée de cellules NK, ou anticorps isolé ou partie de liaison à un antigène de celui-ci pour utilisation selon l'une quelconque des revendications 1 à 22, où :
(a) les cellules NK et l'anticorps ou la partie de liaison à un antigène de celui-ci sont administrés séquentiellement au sujet ;
(b) les cellules NK sont administrées avant l'anticorps ou la partie de liaison à un antigène de celui-ci ;
(c) l'anticorps ou la partie de liaison à un antigène de celui-ci est administré(e) avant les cellules NK ;
(d) les cellules NK et l'anticorps ou la partie de liaison à un antigène de celui-ci sont administrés concurremment dans des compositions séparées ; ou
(e) les cellules NK et l'anticorps ou la partie de liaison à un antigène de celui-ci sont mélangés par admixtion dans une seule composition et administrés concurremment.

24. Kit pour utilisation dans le traitement d'un sujet atteint d'un cancer, le kit comprenant :
(a) une ou plusieurs doses allant d'environ 50 à environ 2000 mg d'un anticorps ou d'une partie de liaison à un antigène de celui-ci qui se lie spécifiquement à CXCR4 exprimé sur la surface d'une cellule cancéreuse ;
(b) une ou plusieurs doses allant d'environ 10⁶ à environ 10¹⁴ d'une population de cellules NKAE ; et
(c) des instructions d'utilisation de l'anticorps ou de partie de celui-ci et des cellules NKAE dans l'utilisation selon l'une quelconque des revendications 2 à 23.
